(19) Europäisches Patentamt
European Patent Office
Office européen des brevets

(11) Veröffentlichungsnummer: **0 149 480**
**B1**

# (12) EUROPÄISCHE PATENTSCHRIFT

(45) Veröffentlichungstag der Patentschrift: **19.04.89**

(21) Anmeldenummer: **85100244.4**

(22) Anmeldetag: **11.01.85**

(51) Int. Cl.⁴: **C 07 F 9/40**, C 07 D 213/74, C 07 C 87/00, C 07 C 91/00, C 07 C 93/04, C 08 K 5/53, C 08 L 75/04, C 08 L 79/04, C 08 G 18/38

## (54) Phosphonsäuresalze.

(30) Priorität: **14.01.84 GB 8401003**
**20.03.84 GB 8407226**
**09.07.84 GB 8417462**
**03.08.84 GB 8419791**

(43) Veröffentlichungstag der Anmeldung: **24.07.85 Patentblatt 85/30**

(45) Bekanntmachung des Hinweises auf die Patenterteilung: **19.04.89 Patentblatt 89/16**

(84) Benannte Vertragsstaaten: **BE CH DE FR GB IT LI NL SE**

(56) Entgegenhaltungen:
**EP-A-0 012 106**
**EP-A-0 057 668**
**EP-A-0 078 478**
**FR-A-2 399 433**
**GB-A-1 324 691**

(73) Patentinhaber: **CIBA- GEIGY AG, Klybeckstrasse 141, CH- 4002 Basel (CH)**

(72) Erfinder: **Clubley, Brian George, 7 Green Villa Park, Wilmslow Cheshire SK9 6EJ (GB)**
Erfinder: **Dellar, Richard John, Dr., 17, Cleveland Road, Hale Altrincham Cheshire WA15 8AY (GB)**
Erfinder: **Buszard, David Lewitt, Dr., 30 Bridle Road, Woodford Cheshire SK7 1QJ (GB)**
Erfinder: **Richardson, Norman, 21 Grey Street, Middleton Manchester M24 3UF (GB)**

(74) Vertreter: **Zumstein, Fritz, Dr., Bräuhausstrasse 4, D-8000 München 2 (DE)**

**EP 0 149 480 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Gegenstand der Erfindung sind Aminsalze von Phosphonsäuren und ihre Verwendung zur flammhemmenderen Ausrüstung von Polyurethanen und Polyisocyanuraten.

Polyurethane und -isocyanurate werden im allgemeinen durch Zusatz einer Phosphor oder Halogen enthaltenden Verbindung oder Mischungen daraus schwerer entflammbar gemacht. Eine hierfür gewöhnlich verwendete phosphorhaltige Verbindung ist das Dimethylmethylphosphonat (DMMP). Mit der Verwendung von DMMP sind jedoch gewisse Schwierigkeiten verbunden. Erstens handelt es sich dabei um eine verhältnismässig leichtflüchtige Flüssigkeit (Siedepunkt 181°C), so dass das Material unter gewissen Umständen durch Verflüchtigung verloren gehen kann. Zweitens wird das DMMP üblicherweise mit zur Herstellung von Polyurethanen und -isocyanuraten zusammen mit Katalysatoren, Treibmitteln und anderen Zusätzen verwendeten Polyolen formuliert, bevor das Isocyanat zugesetzt wird. Die Formulierung neigt zu einer gewissen Unbeständigkeit, wenn sie längere Zeit vor der Verwendung gelagert wird. Unter Umständen nimmt die Viskosität solcher Formulierungen beim Stehenlassen zu. Gegebenenfalls kann man auch unterschiedliche Schäumungscharakteristika beim Vermischen mit dem Isocyanatreaktionspartner feststellen.

Äthanolaminsalze der Methylphosphonsäure sowie deren Verwendung als Zusatzstoffe für hydraulische Flüssigkeiten sind in der europäischen Patentanmeldung 0 012 106 beschrieben.

Aus der EP-A-0 078 478 sind hochfrequenzverschweissbare Polyurethanschaumstoffe und ein Verfahren zur Herstellung derselben bekannt. Zur Überwindung des dielektrischen Verlustfaktors wird vorgeschlagen, dem PU-Material Ammoniumsalze phosphorhaltiger Säuren zuzusetzen. Besonders anzuführen ist dabei das Produkt aus Beispiel 9 und dessen Zusatz zu HF-schweissbaren Weichschaumstoffen gemäss Tabelle 3.

Nicht erkannt und deshalb auch unerwähnt blieb jedoch die Flammfestmachung in dieser Anmeldung.

Als Flammfestmacher ihrerseits sind beispielsweise aus der EP-A-0 057 668 Phosphorverbindungen und dabei insbesondere Guanidinsalze bekannt geworden. Weitere Flammfestmacher auf Basis von Phosphorverbindungen lassen sich der GB-PS-1 324 691 entnehmen. Es werden im wesentlichen Esterverbindungen beschrieben.

Die Aufgabe der vorliegenden Erfindung besteht darin, neue phosphorhaltige Flammschutzmittel geringer Flüchtigkeit bereitzustellen, mittels derer sich lagerstabile Polyolformulierungen erzielen lassen.

Gegenstand der Erfindung sind dementsprechend Aminsalze von Phosphonsäuren der allgemeinen Formel Ia und/oder Ib

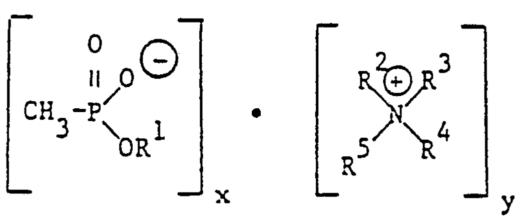

(Ia)

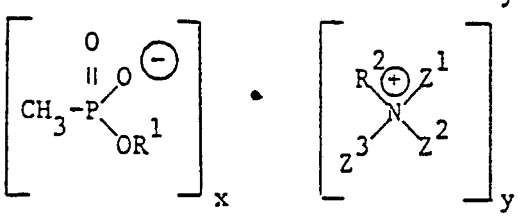

(Ib)

worin

x und y solche ganze Zahlen sind, dass die Anzahl negativer und positiver Ladungen gleich ist, und worin

$R^1$ Wasserstoff, Methyl oder eine negative Ladung bedeutet,

$R^2$ Wasserstoff oder Methyl,

$R^3$ eine durch 1 - 3 gegebenenfalls durch eine Oxyalkylenkette veretherte Hydroxylgruppen substituierte $C_2$-$C_4$-Alkylgruppe bedeuten, wobei sich nicht mehr als eine Hydroxylgruppe an jeweils einem Kohlenstoffatom befindet,

$R^4$ und $R^5$ gleich oder verschieden sein und dieselbe Bedeutung wie $R^3$ haben können oder Wasserstoff, eine $C_1$-$C_4$-Alkyl-, Phenyl-, Benzylgruppe oder eine am aromatischen Ring durch eine $C_1$-$C_{12}$-Alkylgruppe, eine gegebenenfalls durch eine Oxyalkylenkette veretherte Hydroxylgruppe und/oder 1 - 3 Halogenatome substituierte Phenyl- oder Benzylgruppe bedeuten oder

$R^5$ eine Gruppe der Formel II

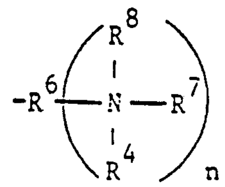 (II)

bedeutet, worin

$R^6$ eine $C_2$-$C_4$-Alkylen-, Phenylen-, Xylylen- oder Diphenylmethangruppe oder eine Gruppe der Formel

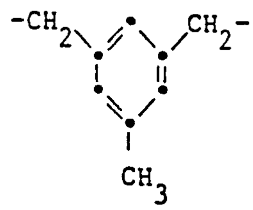 oder 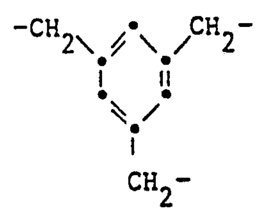

ist, wobei aromatische Ringe in einer $R^6$-Gruppe gegebenenfalls durch eine $C_1$-$C_{12}$-Alkylgruppe, eine gegebenenfalls durch eine Oxyalkylenkette veretherte Hydroxylgruppe und/oder 1 - 3 Halogenatome substituiert sein können, und

$R^7$ Wasserstoff oder Methyl bedeutet, wobei das Stickstoffatom eine positive Ladung trägt, oder $R^7$ nicht vorhanden ist;

$R^8$ Wasserstoff ist oder dieselbe Bedeutung wie $R^3$ hat und

n 1 oder 2 bedeutet

oder $R^4$ und $R^5$ gegebenenfalls zusammen mit dem Stickstoffatom einen gegebenenfalls ein Sauerstoffatom enthaltenden 5- oder 6-gliedrigen heterocyclischen Ring bilden; sowie

$Z^1$, $Z^2$ und $Z^3$ gleich oder verschieden sind und Wasserstoff, eine gerad- oder verzweigtkettige $C_1$-$C_{12}$-Alkyl- bzw. $C_3$-$C_{12}$-Alkenyl- oder Alkinylgruppe, eine $C_4$-$C_{12}$-Cycloalkylgruppe, eine gegebebenenfalls durch eine gerad- oder verzweigtkettige $C_1$-$C_4$-Alkylgruppe, eine $C_1$-$C_4$-Alkoxygruppe, Amino, Methylamino, Halogen oder Nitro substituierte Phenyl- oder Naphthylgruppe oder eine $C_7$-$C_{12}$-Aralkylgruppe bedeuten oder

$Z^2$ und $Z^3$ zusammen mit dem sie verknüpfenden Stickstoffatom ein gesättigtes oder ungesättigtes 3- bis 7-gliedriges Ringsystem bilden, welches gegebenenfalls ein weiteres Heteroatom enthalt und gegebenenfalls durch eine gerad- oder verzweigtkettige $C_1$-$C_4$-Alkylgruppe, eine $C_1$-$C_4$-Alkoxygruppe, Amino, Methylamino, eine $C_1$-$C_4$-Aminoalkylgruppe, Halogen oder Nitro substituiert sein kann,

oder $Z^1$, $Z^2$ und $Z^3$ zusammen mit dem sie verknüpfenden Stickstoffatom einen gegebenenfalls ein weiteres Heteroatom enthaltenden 8- bis 12-gliedrigen bicyclischen Ring bilden, oder

$Z^1$ eine Gruppe der Formel

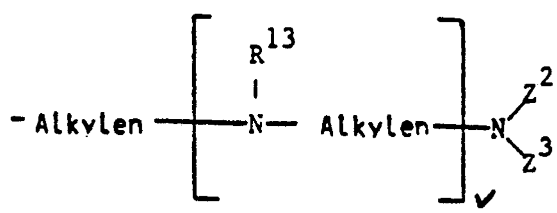

darstellt, worin

Alkylen eine Gruppe mit 2 - 12 Kohlenstoffatomen,

v 0 oder eine ganze Zahl von 1 - 5,

$R^{13}$ Wasserstoff oder eine gerad- bzw. verzweigtkettige $C_1$-$C_{16}$-Alkylgruppe bedeuten und

$Z^2$ sowie $Z^3$ dieselbe Bedeutung wie obenstehend definiert besitzen,

oder $Z^1$ eine Gruppe der Formel

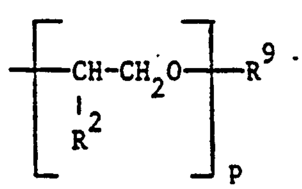 oder 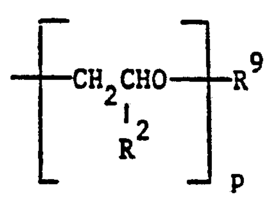

ist, worin

$R^2$ die oben angegebene Bedeutung hat und

$R^9$ eine $C_1$-$C_{12}$-Alkylgruppe und

p eine ganze Zahl von 1 bis 10, vorzugsweise 1 - 4 bedeuten; oder $Z^3$ eine Gruppe der Formel IIa

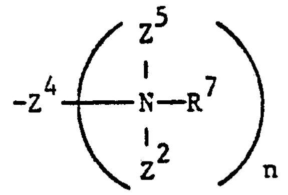

(IIa)

darstellt, worin

$Z^4$ eine $C_2$-$C_{12}$-Alkylengruppe, eine Phenylen-, Xylylen- oder Diphenylmethangruppe oder eine Gruppe der Formel

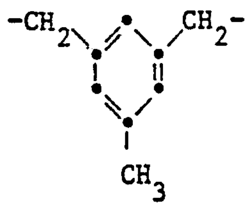

oder

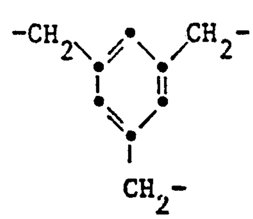

bedeutet, wobei aromatische Ringe in einer $Z^4$-Gruppe gegebenenfalls durch eine $C_1$-$C_{12}$-Alkylgruppe, Hydroxyl und/oder 1 - 3 Halogenatome substituiert sein können, und

$R^7$ Wasserstoff oder Methyl bedeutet, wobei das Stickstoffatom eine positive Ladung trägt, oder $R^7$ nicht vorhanden ist;

$Z^5$ Wasserstoff oder eine Gruppe gemäss Definition für $Z^1$ ist und

n 1 oder 2 bedeutet;

oder $Z^2$ und $Z^5$ mit dem Stickstoffatom einen gegebenenfalls ein weiteres Heteroatom enthaltenden 3- bis 7-gliedrigen heterocyclischen Ring bilden können,

mit der Massgabe, dass, wenn das Salz der Formel Ia nicht vorhanden ist und $R^1$ Wasserstoff oder eine negative Ladung, $R^2$ Wasserstoff und zwei der Reste $Z^1$, $Z^2$ und $Z^3$ Wasserstoff bedeuten, der andere Rest weder Alkyl noch Alkenyl mit mehr als 7 Kohlenstoffatomen sein kann

und mit der Massgabe, dass Verbindungen mit den Formeln

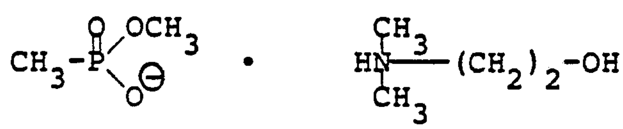

und

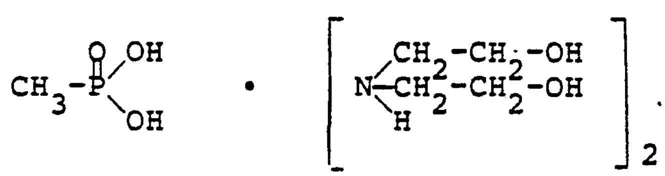

ausgenommen sind.

Sofern die Hydroxylgruppen in $R^3$, $R^4$ und $R^5$ durch eine Oxyalkylenkette verethert sind, kann das Kettenglied die Formel

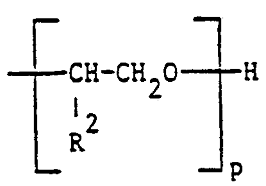

oder

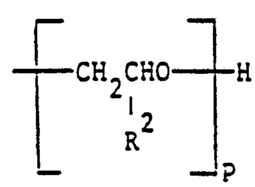

aufweisen, worin $R^2$ die zuvor genante Bedeutung hat und p eine ganze Zahl von 1 bis 10, vorzugsweise 1 - 4 ist.

$R^3$, $R^4$ oder $R^5$ als eine durch 1 bis 3 Hydroxylgruppen substituierte $C_2$-$C_4$-Alkylgruppe kann beispielsweise 2-Hydroxyäthyl, 2-Hydroxy-n-propyl oder Trimethylolmethyl sein.

Für $R^4$ oder $R^5$ als eine $C_1$-$C_4$-Alkylgruppe kommen beispielsweise Methyl, Äthyl, Isopropyl, n-Butyl oder sek.-Butyl in Frage.

Wenn es sich bei $R^4$ oder $R^5$ um eine am aromatischen Ring durch eine Alkylgruppe mit 1 - 12

Kohlenstoffatomen substituierte Phenyl- oder Benzylgruppe handelt, so kommen als solche Alkylgruppe zum Beispiel Methyl, Äthyl, Isopropyl, n-Butyl, t-Butyl, n-Hexyl, n-Octyl, n-Nonyl oder Octadecyl in Betracht.

Weitere nicht-einschränkende Beispiele für $R^3$, $R^4$ und $R^5$ finden sich in der Liste der Amine der Formel IV, die zur Herstellung der Salze der Formel Ia verwendet werden können.

$Z^1$, $Z^2$ und $Z^3$ können die folgenden Bedeutungen besitzen: zu $C_1$-$C_{12}$-Alkylgruppen zählen Methyl, Äthyl, Isopropyl, n-Butyl, sek.-Butyl, Octyl und Dodecyl; die $C_3$-$C_{12}$-Alkenylgruppen können Allyl, Propenyl, Butenyl, Hexenyl oder Dodecenyl bedeuten; die $C_3$-$C_{12}$-Alkinylgruppen schliessen den Propargylrest ein; als $C_4$-$C_{12}$-Cycloalkylgruppen kommen Cyclobutyl, -pentyl, -hexyl, -octyl und -dodecyl in Betracht; die Aralkylgruppen können Benzyl, Phenäthyl oder 1-Naphthylmethyl bedeuten; gegebenenfalls substituierte Arylgruppen umfassen die Phenyl-, Methoxyphenyl-, Aminophenyl-, Nitrophenyl-, Chlorphenyl- und Naphthylgruppe; als Aminoalkylgruppen kommen Aminoäthyl und Aminopropyl in Betracht.

Falls $Z^2$ und $Z^3$ zusammen mit dem Stickstoffatom, an das sie gebunden sind, ein 3- bis 7-gliedriges Ringsystem bilden, so kommen dafür beispielsweise Pyridino, Morpholino, Piperidino, Piperazino, 2-Aminopyridino, 2-Methoxypyridino oder 3-Chlorpyridino in Betracht.

Weitere nicht-einschränkende Beispiele für $Z^1$, $Z^2$ und $Z^3$ finden sich in der Liste der Amine der Formel V, die zur Herstellung der Salze der Formel Ib verwendet werden können.

Bevorzugt werden Salze der Formel Ia

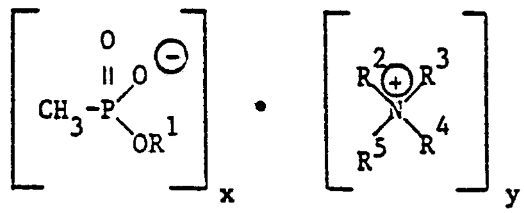

(Ia)

worin x, y, $R^1$, $R^2$, $R^3$, $R^4$ und $R^5$ die oben angegebene Bedeutung haben.

Weiterhin werden auch Aminsalze der Formel Ib bevorzugt:

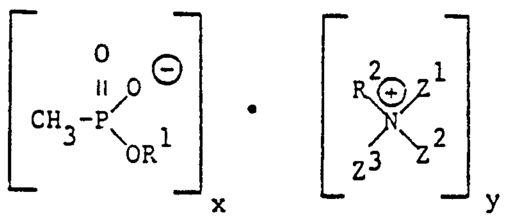

(Ib)

worin x, y, $R^1$, $R^2$, $Z^1$, $Z^2$ und $Z^3$ die oben angegebenen Bedeutungen haben.

$R^1$ ist vorzugsweise Methyl.

In der Formel Ia steht $R^2$ vorzugsweise für Methyl sowie $R^3$ und $R^4$ für Hydroxyäthyl.

In den Formeln Ia und Ib sind $R^1$ und $R^2$ vorzugsweise Methyl.

Die erfindungsgemässen Aminsalze lassen sich dadurch herstellen, dass man Methylphosphonsäure bzw. deren Ester der Formel III

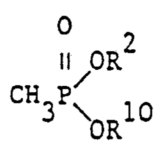

(III)

worin

$R^2$ die oben angegebene Bedeutung hat und

$R^{10}$ für Wasserstoff oder Methyl steht, mit einem Amin der Formel IV und/oder V

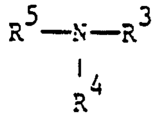

(IV)

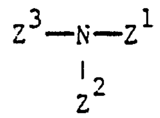

(V)

worin $R^3$, $R^4$, $R^5$, $Z^1$, $Z^2$ und $Z^3$ die oben angegebenen Bedeutungen haben, umsetzt. Nach dem Verfahren gemäss vorliegender Erfindung werden die Aminsalze in einem wässrigen oder organischen Lösungsmittel sowie gegebenenfalls in einer Inertgasatmosphäre, und nötigenfalls unter Erhitzen, um die Salzbildung zu veranlassen, umgesetzt.

Der Anteil der Methylphosphonsäure oder -ester der Formel III wird so bemessen, dass sie mit einer oder mehreren Amingruppen im Amin der Formel IV oder V in der Weise reagiert, dass je nach Wunsch das Mono-, Di- oder ein höherwertiges Salz entsteht.

Im Falle der Verwendung von Methylphosphonsäure oder ihres Monomethylesters tritt eine schnelle Salzbildung ein, so dass milde Reaktionsbedingungen herrschen können. Die Säure und das Amin können einfach bei Raumtemperatur miteinander zur Salzbildung vermischt werden. Falls das Amin eine viskose Flüssigkeit oder einen Feststoff darstellt, kann das Gemisch z. B. bis auf 100°C erhitzt werden, um eine wirkungsvolle Reaktion zu erzielen. Je nach Bedarf kann ein wässriges oder organisches Lösungsmittel eingesetzt werden, das nach Beendigung der Reaktion entfernt wird.

Im Falle der Verwendung von DMMP sind strengere Reaktionsbedingungen zur Erzielung einer vollständigen Salzbildung notwendig. Das DMMP/Amingemisch kann bis auf 180°C gegebenenfalls unter Inertgasatmosphäre wie z. B. Stickstoff erhitzt werden. Das Erhitzen auf 100°C - 180°C kann u.U. mehrere Stunden erfordern. Die Reaktion kann gewünschtenfalls in einem organischen Lösungsmittel durchgeführt werden, beispielsweise in einem Kohlenwasserstofflösungsmittel wie z. B. Toluol oder Xylol.

Die erfindungsgemäss eingesetzten Stoffe, d. h. Methylphosphonsäure und ihre Ester sowie die Amine, sind sämtlich bekannte Verbindungen und können in bekannter Weise hergestellt werden.

Wenn Produkte mit einem Gehalt an Oxyalkylenketten hergestellt werden, können diese entweder durch Reaktion des Amins mit einem Alkylenoxyd unter nachfolgender Salzbildung eingeführt werden, oder man lässt das Amin unter Salzbildung reagieren und bringt es daran anschliessend mit einem Alkylenoxyd zur Reaktion. Die Verätherung kann gewünschtenfalls vor oder nach der Salzbildung durchgeführt werden. Gegebenenfalls können 1 - 10 Mole Äthylen- und/oder Propylenoxyd pro Mol Amin oder Salz eingesetzt werden. Man kann die Alkoxylierung bei einer Temperatur von 30°C - 200°C in Anwesenheit oder Abwesenheit eines Katalysators durchführen.

Erfindungsgemäss einsetzbare Amine der Formel IV mit einem Gehalt an Oxyalkylenketten schliessen solche der Formeln

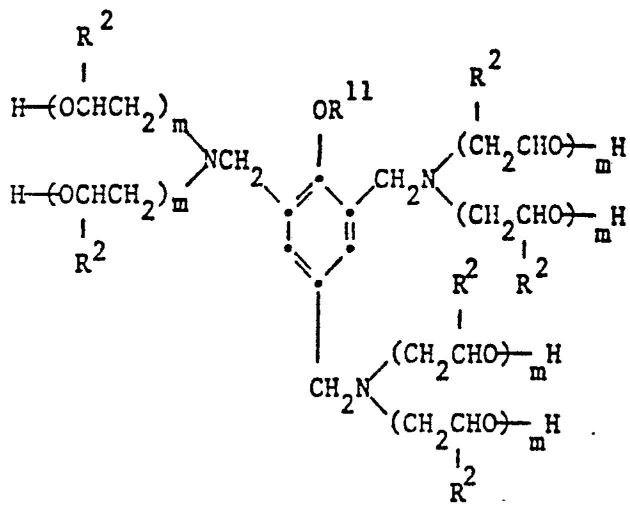

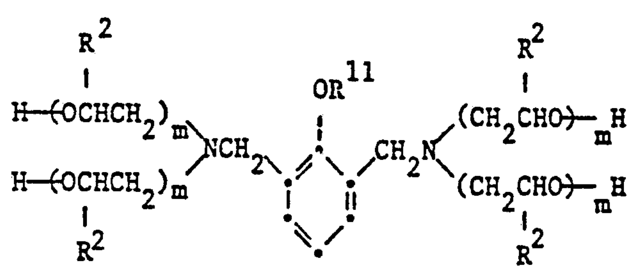

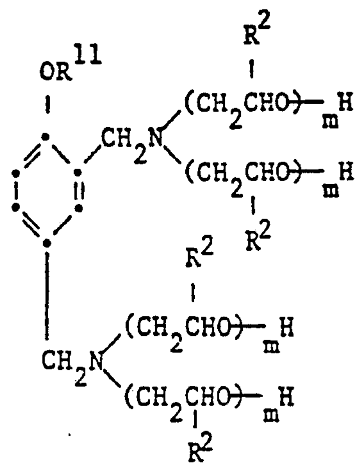

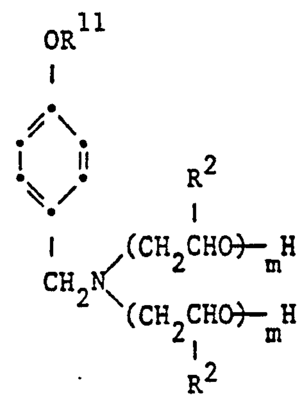

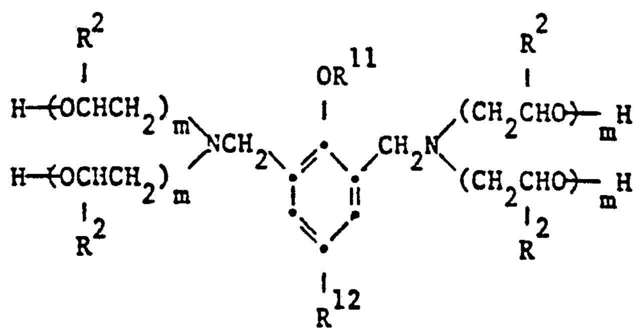

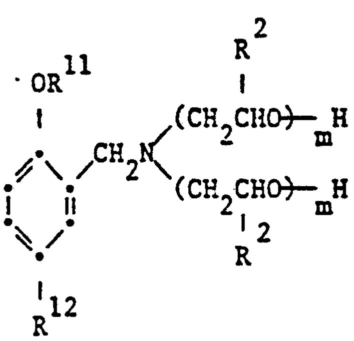

ein, worin

$R^2$ Wasserstoff oder Methyl bedeutet und im Molekül jeweils gleich oder verschieden sein kann,

$R^{11}$ Wasserstoff, $-(CH_2CH_2O-)_qH$ oder

$-(CH_2\underset{\displaystyle CH_3}{\underset{|}{C}}HO-)-_qH$ ist, wobei q 1 - 4 bedeutet,

$R^{12}$ eine $C_1$-$C_{12}$-Alkylgruppe, z. B. eine Nonylgruppe bedeutet und m jeweils gleich oder verschieden ist und 1 - 4 bedeutet.
Beispiele für verwendbare Amine der Formel IV sind die folgenden:

$H_2NCH_2CH_2OH$ $\qquad$ $HN(CH_2CH_2OH)_2$

$N(CH_2CH_2OH)_3$ $\qquad$ $HN(CH_2\underset{\displaystyle CH_3}{\underset{|}{C}}HOH)_2$

$N(CH_2\underset{\displaystyle CH_3}{\underset{|}{C}}HOH)_3$ $\qquad$ $CH_3N(CH_2CH_2OH)_2$

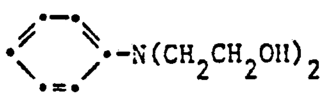 $\qquad$ $H_2N-C(CH_2OH)_3$

$(CH_3)_2NCH_2CH_2OH$ $\qquad$ $CH_3NHCH_2CH_2OH$

$(HOCH_2CH_2)_2NCH_2CH_2N(CH_2CH_2OH)_2$ $\qquad$ $(HO\underset{\displaystyle CH_3}{\underset{|}{C}}HCH_2)_2NCH_2CH_2N(CH_2\underset{\displaystyle CH_3}{\underset{|}{C}}HOH)_2$

Äthylendiamin/Alkylenoxydkondensationsprodukte wie solche, die unter dem Handelsnamen Propylan A260® (Diamond Shamrock) im Handel sind.

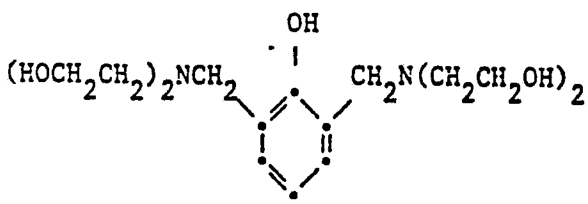

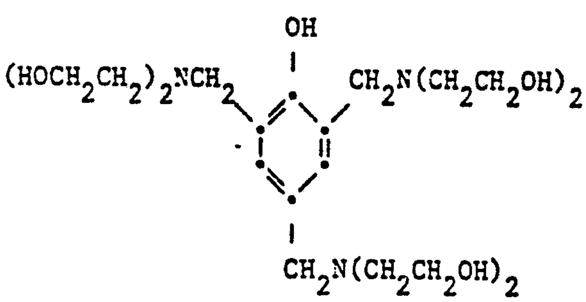

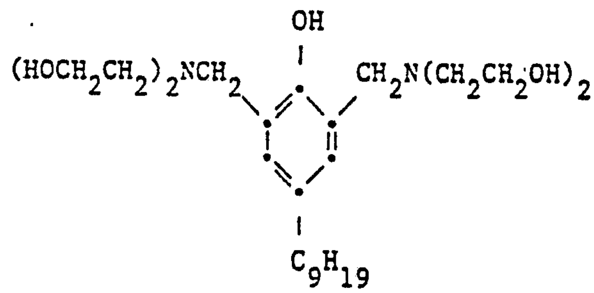

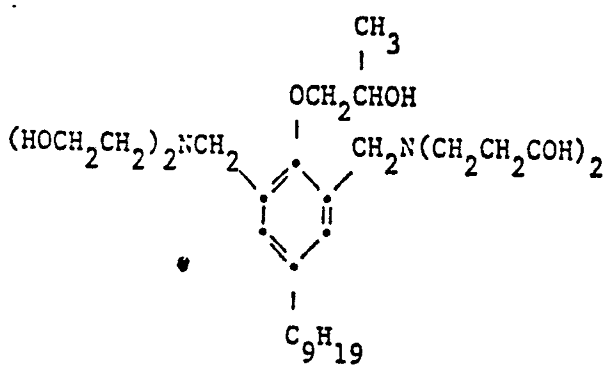

Phenol/Formaldehyd/Diäthanolamin-Mannichbasen, in welche Alkylenoxydgruppen eingeführt wurden, wie z. B. solche, welche unter dem Warenzeichen Thanol R650x® oder Thanol R350x® (Texaco) gehandelt werden und in US-A-3 297 597 beschrieben sind.

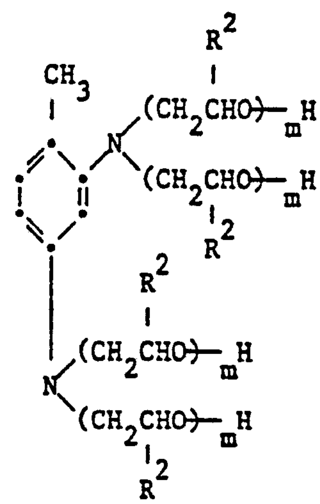

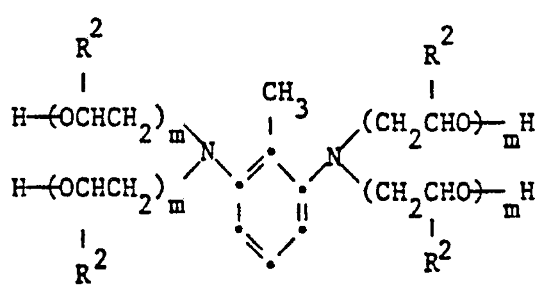

worin $R^2$ die oben angegebene Bedeutung hat,

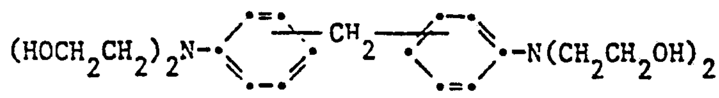

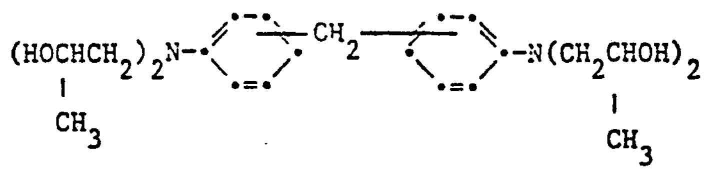

sowie ihre Gemische mit Oligomeren der Formel

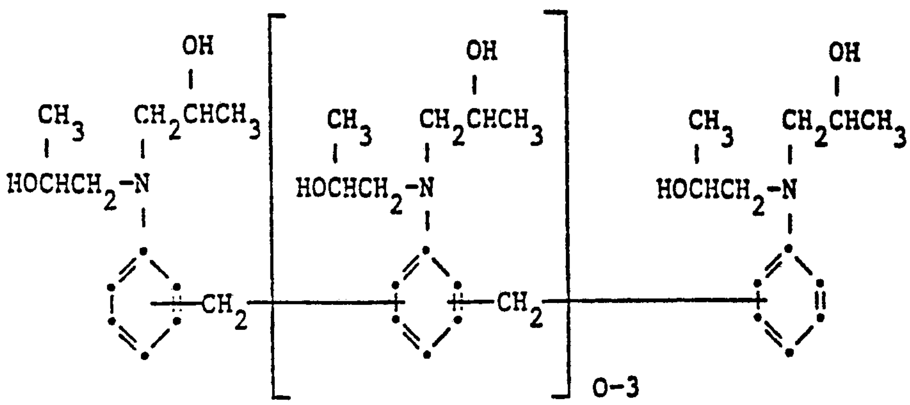

Zur Herstellung der erfindungsgemässen Salze geeignete Amine der Formel V umfassen: Methylamin, Dimethylamin, Trimethylamin, Triäthylamin, Propylamin, n-Butylamin, sek.-Butylamin, Tri-n-butylamin, Dodecylamin, Allylamin, Diallylamin, Propargylamin, Cyclobutylamin, Cyclopentylamin, Cyclohexylamin, Cyclooctylamin, Anilin, o-Anisidin, p-Anisidin, o-Phenylendiamin, p-Phenylendiamin, N-Methylanilin, o-Nitroanilin, o-Chloranilin, α-Naphthylamin, N,N-Dimethylanilin, Benzylamin, Phenäthylamin, 1-Naphthylmethylamin, Pyridin, Morpholin, Piperidin, Piperazin, 2-Aminopyridin, 2-Methoxypyridin, 3-Chlorpyridin, Äthylenimin, Chinuclidin, 1,4-Diazabicyclo[2.2.2]octan, Äthylendiamin, Diäthylentriamin, Triäthylentetramin, Hexamethylendiamin, Dodecamethylendiamin und 2,4,6-Tris-(dimethylaminomethyl)-phenol.

Man kann die erfindungsgemässen Aminsalze allein oder zusammen mit anderen Flammschutzmitteln einsetzen wie z. B. solchen, welche untenstehend als Zusätze zum Zweck der flammschutzhemmenden Ausrüstung von harten oder elastischen Polyurethanen oder Polyisocyanuraten aufgezählt sind, wobei man jene durch Reagierenlassen eines Polyols mit einem Polyisocyanat in Anwesenheit eines Treibmittels, sofern ein Schaumstoff produziert werden soll, und in Anwesenheit eines Katalysators herstellt. Der Anteil an Polyisocyanat variiert je nach Art des herzustellenden Produkts. Der Anwendungsbereich der vorliegenden Erfindung erstreckt sich über den gesamten Polymerbereich mit einem Isocyanatindex von 1 - 6, vorzugsweise von 1 - 4,5.

Gegenstand der Erfindung ist weiterhin ein Polyurethan oder Polyisocyanurat mit einem darin eingelagerten flammhemmenden Aminsalz der Formel Ia und/oder Ib

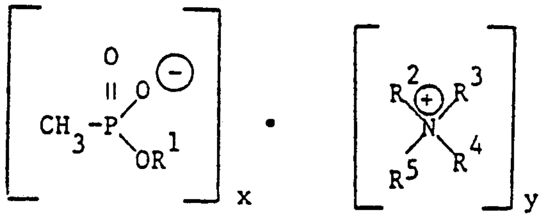

(Ia)

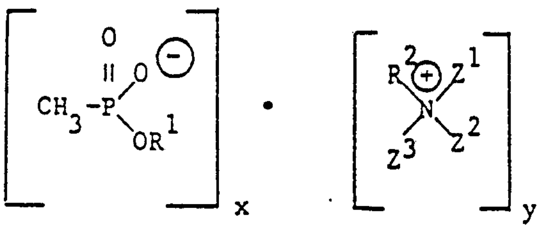

(Ib)

worin

x und y solche ganze Zahlen sind, dass die Anzahl negativer und positiver Ladungen gleich ist, und worin $R^1$ Wasserstoff, Methyl oder eine negative Ladung bedeutet,

$R^2$ Wasserstoff oder Methyl,

$R^3$ eine durch 1 - 3 gegebenenfalls durch eine Oxyalkylenkette veretherte Hydroxylgruppen substituierte $C_2$-$C_4$-Alkylgruppe bedeuten, wobei sich nicht mehr als eine Hydroxylgruppe an jeweils einem Kohlenstoffatom befindet,

$R^4$ und $R^5$ gleich oder verschieden sein und dieselbe Bedeutung wie $R^3$ haben können oder Wasserstoff, eine $C_1$-$C_4$-Alkyl-, Phenyl-, Benzylgruppe oder eine am aromatischen Ring durch eine $C_1$-$C_{12}$-Alkylgruppe, eine gegebenenfalls durch eine Oxyalkylenkette veretherte Hydroxylgruppe und/oder 1 - 3 Halogenatome substituierte Phenyl- oder Benzylgruppe bedeuten oder

$R^5$ eine Gruppe der Formel II

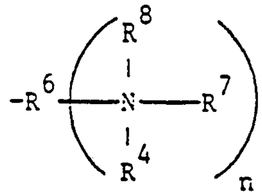

(II)

bedeutet, worin

$R^6$ eine $C_2$-$C_4$-Alkylen-, Phenylen-, Xylylen- oder Diphenylmethangruppe oder eine Gruppe der Formel

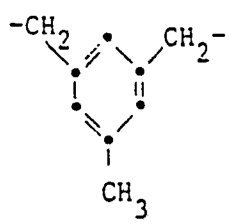

oder

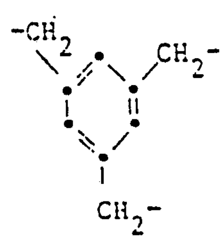

ist, wobei aromatische Ringe in einer $R^6$-Gruppe gegebenenfalls durch eine $C_1$-$C_{12}$-Alkylgruppe, eine gegebenenfalls durch eine Oxyalkylenkette veretherte Hydroxylgruppe und/oder 1 - 3 Halogenatome substituiert sein können, und

$R^7$ Wasserstoff oder Methyl bedeutet, wobei das Stickstoffatom eine positive Ladung trägt, oder $R^7$ nicht vorhanden ist;

$R^8$ Wasserstoff ist oder dieselbe Bedeutung wie $R^3$ hat und

n 1 oder 2 bedeutet oder

$R^4$ und $R^5$ gegebenenfalls zusammen mit dem Stickstoffatom einen gegebenenfalls ein Sauerstoffatom enthaltenden 5- oder 6-gliedrigen heterocyclischen Ring bilden; sowie

$Z^1$, $Z^2$ und $Z^3$ gleich oder verschieden sind und Wasserstoff, eine gerad- oder verzweigtkettige $C_1$-$C_{12}$-Alkyl- bzw. $C_3$-$C_{12}$-Alkenyl- oder Alkinylgruppe, eine $C_4$-$C_{12}$-Cycloalkylgruppe, eine gegebenenfalls durch eine gerad- oder verzweigtkettige $C_1$-$C_4$-Alkylgruppe, eine $C_1$-$C_4$-Alkoxygruppe, Amino, Methylamino, Halogen oder Nitro substituierte Phenyl- oder Naphthylgruppe oder eine $C_7$-$C_{12}$-Aralkylgruppe bedeuten

oder $Z^2$ und $Z^3$ zusammen mit dem sie verknüpfenden Stickstoffatom ein gesättigtes oder ungesättigtes 3- bis 7-gliedriges Ringsystem bilden, welches gegebenenfalls ein weiteres Heteroatom enthält und

gegebenenfalls durch eine gerad- oder verzweigtkettige $C_1$-$C_4$-Alkylgruppe eine $C_1$-$C_4$-Alkoxygruppe, Amino, Methylamino, eine $C_1$-$C_4$-Aminoalkylgruppe, Halogen oder Nitro substituiert sein kann,

oder $Z^1$, $Z^2$ und $Z^3$ zusammen mit dem sie verknüpfenden Stickstoffatom einen gegebenenfalls ein weiteres Heteroatom enthaltenden 8- bis 12-gliedrigen bicyclischen Ring bilden,

oder $Z^1$ eine Gruppe der Formel

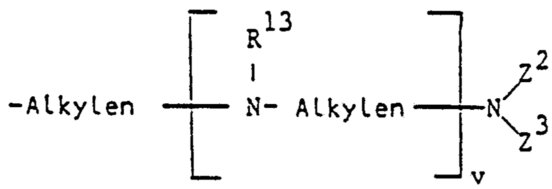

darstellt, worin

Alkylen eine Gruppe mit 2 - 12 Kohlenstoffatomen

v 0 oder eine ganze Zahl von 1 - 5,

$R^{13}$ Wasserstoff oder eine gerad- bzw. verzweigtkettige $C_1$-$C_{16}$-Alkylgruppe bedeuten und

$Z^2$ sowie $Z^3$ dieselbe Bedeutung wie obenstehend definiert besitzen,

oder $Z^1$ eine Gruppe der Formel

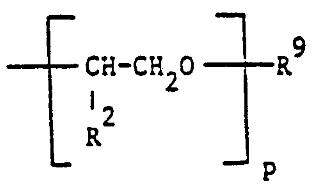

oder

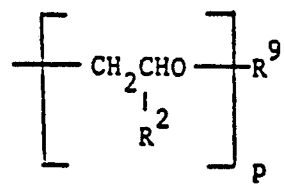

ist, worin

$R^2$ die oben angegebene Bedeutung hat und

$R^9$ eine $C_1$-$C_{12}$-Alkylgruppe und

p eine ganze Zahl von 1 bis 10, vorzugsweise 1 - 4 bedeuten;

oder $Z^3$ eine Gruppe der Formel IIa

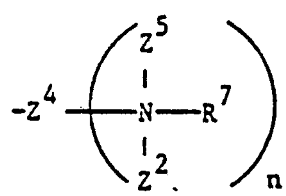

(IIa)

darstellt, worin

$Z^4$ eine $C_2$-$C_{12}$-Alkylengruppe, eine Phenylen-, Xylylen- oder Diphenylmethangruppe oder eine Gruppe der Formel

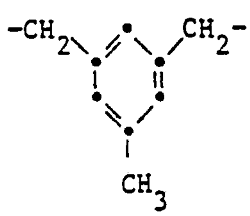

oder

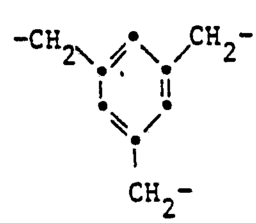

bedeutet, wobei aromatische Ringe in einer $Z^4$-Gruppe gegebenenfalls durch eine $C_1$-$C_{12}$-Alkylgruppe, Hydroxyl und/oder 1 - 3 Halogenatome substituiert sein können, und

$R^7$ Wasserstoff oder Methyl bedeutet, wobei das Stickstoffatom eine positive Ladung trägt, oder $R^7$ nicht vorhanden ist;

$Z^5$ Wasserstoff oder eine Gruppe gemäss Definition für $Z^1$ ist und

n 1 oder 2 bedeutet;

oder $Z^2$ und $Z^5$ mit dem Stickstoffatom einen gegebenenfalls ein weiteres Heteroatom enthaltenden 3- bis 7-gliedrigen heterocyclischen Ring bilden können mit der Massgabe, dass die Verbindung mit der Formel

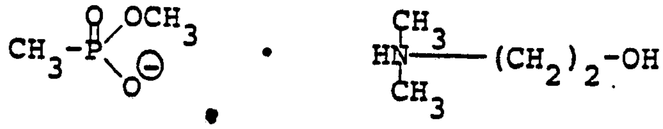

für Polyurethane ausgenommen ist.

Falls ein von einem Polyol abgeleitetes Aminsalz verwendet wird, kann man jenes allein als die einzige Polyolkomponente für die Reaktion mit einem Polyisocyanat zur Erzielung eines Polyurethans oder Polyisocyanurats einsetzen. In den meisten Fällen, wo eine solch hohe Flammschutzmittelmenge nicht nötig ist, kann man die Salze in das Polyurethan oder Polyisocyanurat in der Weise einbringen, dass man sie dem zur Herstellung des Polyurethans oder Polyisocyanurats verwendeten Reaktionsgemisch hinzufügt.

Man kann die erfindungsgemässen Aminsalze auch alternativ zum Hydroxylgruppen (Polyester oder Polyätherpolyol) enthaltenden Reaktionspartner oder dessen Reaktion mit dem Polyisocyanat hinzufügen. Wenn man hydroxylgruppenhaltige Aminsalze verwendet, werden diese normalerweise mit dem Isocyanat ebenso wie das Polyol reagieren. Die Viskosität des Polyol/Salzgemisches ändert sich bei Lagerung bei Raumtemperatur nicht nennenswert. Man erzielt gleichmässige Aufschäumungsbedingungen, welche sich beim Lagern nicht verändern.

Alternativ kann man ein Präpolymer durch Reaktion eines Aminsalzes der Formel Ia und Ib mit einem Polyisocyanat zur Herstellung eines Produkts mit endständigen Isocyanat- oder Hydroxylgruppen gewinnen, wobei anschliessend das Polymer durch weitere Reaktion mit dem Polyol/Polyisocyanat hergestellt wird.

Der Anteil des in die Polyurethane oder Polyisocyanurate einführbaren erfindungsgemässen flammhemmenden Aminsalzes hängt vom Grad des erforderlichen Flammschutzes ab. Der Anteil des flammhemmenden Salzes kann im Falle des gleichzeitigen Einsatzes eines Polyols typischerweise 1 - 200, vorzugsweise 3 - 100 Gew.-Teile, bezogen auf 100 Gew.-Teile Polyol, betragen.

Man kann die Aminsalze zusammen mit anderen flammhemmenden Verbindungen mischen. Als Beispiele dafür wären u. a. Halogen enthaltende Verbindungen wie z. B. aliphatische und aromatische Bromverbindungen, oxyalkylierte Phosphatester, Chloralkylphosphate und -phosphonate oder Triarylphosphate zu erwähnen. Weitere Beispiele brauchbarer Verbindungen sind der Pentabromdiphenyl- oder Dibromkresylglycidyläther, Tetrabrombisphenol A, Dibromneopentylglykol, ein mittels Reaktion von Tetrabromphthalsäureanhydrid mit Äthylen- und/oder Propylenoxyd hergestelltes Diol, Tris-(chloräthyl)-phosphat, Tris-(monochlorpropyl)-phosphat, Diäthyl-bis-(hydroxyäthyl)-aminomethylphosphonat sowie isopropylierte oder t-butylierte Phenylphosphatgemische, wie sie aus der GB-A-1 146 173 bekannt sind, Trikresylphosphat, Trixylylphosphat und Kresyldiphenylphosphat. Das Verhältnis der Aminsalze zu anderen flammhemmenden Verbindungen kann zwischen 5 : 95 und 95 : 5 betragen.

Gegebenenfalls können die Polyurethan- und Polyisocyanuratzusammensetzungen auch Methylphosphonsäure und ihre Mono- und Dimethylester enthalten, welche nicht mit dem vorliegenden Amin in Reaktion getreten sind, obschon saure Zusätze vorzugsweise nicht in der Formulierung vorhanden sind.

Die zur Herstellung von Polyurethanen oder Polyisocyanuraten verwendeten Isocyanate und Polyole können jede der aus dem Stand der Technik bekannten Verbindungen sein.

Das Isocyanat liegt gewöhnlich in flüssiger Form, wie Toluoldiisocyanat, gegebenenfalls hydriertes oder polymeres Methylendiphenyldiisocyanat, Hexamethylendiisocyanat, Isophorondiisocyanat und als ein beliebiges Polyisocyanatpräpolymer mit zwei oder mehr unumgesetzten Isocyanatresten und dgl. vor. Üblicherweise enthält das erfindungsgemäss verwendete Toluoldiisocyanat die Isomeren 2,4- und 2,6-Toluoldiisocyanat. Der Isomeranteil ist jedoch nicht kritisch.

Das zur Hartschaumstoffbildung benutzte Polyol kann eine aus der Reaktion einer Polyhydroxylverbindung wie z. B. Glyzerin, Sucrose, Sorbit, Trimethylpropan, Pentaerythrit, Triäthanolamin oder einem Amin, wie z. B. einem Äthylendiamin, polyaromatischem Amin oder eine aromatischen Mannichbase mit Propylen- und/oder Äthylenoxyd erhaltene polyfunktionelle aktive Wasserstoffverbindung sein.

Im allgemeinen sind die für die Herstellung von elastischen Polyurethanschaumstoffen verwendeten Polyole Polyätherpolyole wie z. B. Polyoxyäthylen/Oxypropylendiole bzw. -triole, Rizinusöl und Methylglucosidpolyätherpolyole mit einem mittleren Molekulargewichtsbereich von ca. 250 - 6500. Andere anstelle von Polyätherpolyolen verwendbare Polyole sind Polyesterpolyole wie z. B. die Reaktionsprodukte einer aliphatischen bifunktionellen Carbonsäure wie z. B. Adipin- oder Sebacinsäure mit einer zwei- oder dreifach funktionellen Hydroxyverbindung wie z. B. Äthylen-, Diäthylen-, Propylen- oder 1,4-Butylenglykol und Butantriol.

Gegebenenfalls können Polyole wie z. B. Glyzerin, Hexan- oder Butantriol, Trimethylpropan oder -äthan und Pentaerythrit bei der Polymerisationsreaktion mit dem Polyol zur Aufrechterhaltung eines gewünschten stöchiometrisch ausgewogenen Verhältnisses des Isocyanats gegenüber dem Hydroxylanteil miteingesetzt werden.

Bei der Herstellung von Polyurethan- und Polyisocyanuratschaumstoffen können beliebige übliche basische Katalysatoren wie z. B. Natriumhydroxyd, Natriumacetat, tertiäre Amine oder Stoffe mit der Eigenschaft zur Bildung tertiärer Amine wie z. B. Trimethylamin, Triäthylendiamin, N-Methylmorpholin, N,N-Dimethylcyclohexylamin und N,N-Dimethylaminoäthanol eingesetzt werden. Man kann ebenfalls

Metallverbindungen wie z. B. Kohlenwasserstoff-Zinnalkylcarboxylate, Dibutylzinndiacetat, Dibutylzinndioctoat oder -dilaurat und Zinn(II)octoat verwenden. In gleicher Weise sind zum Auslösen der Trimerisierung von Isocyanat vorgesehene Verbindungen, beispielsweise 2,4,6-Tris-(N,N-dimethylaminomethyl)-phenol, 1,3,5-Tris-(N,N-dimethyl-3-aminopropyl)-S-hexahydrotriazin, Kaliumoctoat oder -acetat und Katalysatoren, wie jene, die unter dem Warenzeichen DABCO TMR® und POLYCAT 43® gehandelt werden, verwendbar.

Zahlreiche andere Katalysatoren können gewünschtenfalls anstelle der oben aufgelisteten treten. Der Anteil an Katalysator kann im Bereich von ca. 0,05 bis ca. 5 Gew.-% oder darüber liegen, wobei das Gewicht auf das Gesamtgewicht der (des) eingesetzten Polyole (Polyols) bezogen ist. Es können auch Gemische der oben genannten und/oder anderer Katalysatoren verwendet werden.

Um den Polyol/Polyisocyanatgemischen eine geschäumte oder zellige Struktur zu verleihen, muss ein geeignetes Treibmittel oder ein entsprechendes Treibmittelsystem hinzugefügt oder in situ erzeugt werden. Geeignete Treibmittel schliessen die flüssigen aber verhältnismässig flüchtigen halogenierten Kohlenwasserstoffe wie z. B. Dichlordifluormethan, Trichlorfluormethan und Methylenchlorid ein. Diese werden in flüssiger Form in Mengen von ungefähr 5 bis ca. 50 Gew.-%, bezogen auf das Polyolgewicht, einer oder mehrerer Komponenten des Polyol/Polyisocyanatgemischs hinzugefügt und grösstenteils in der flüssigen Mischung zur Zellbildung verflüchtigt. Die Mischung härtet anschliessend zu ihrer endgültigen zelligen Struktur aus.

Obschon die halogenierten Kohlenwasserstoffe besonders dann wünschenswerte Treibmittel darstellen, wenn aussergewöhnliche isolierende Eigenschaften gefragt sind, können andere Treibmittel wie z. B. Kohlendioxyd, welches durch Zusatz von Wasser zum Polyol oder gleichzeitig mit dem Zusatz von Polyisocyanat erzeugt wird, insbesondere für elastische offenzellige Schaumstoffe angewendet werden.

Es sei auch noch angemerkt, dass das Aufschäumen gegebenenfalls auch durch die kombinierte Verwendung eines Treibmittels in Verbindung mit Wasserzusatz zum Polyol erzielbar ist.

Zur Erzielung einer verhältnismässig gleichförmigen Verteilung der verschiedenen Komponenten des flüssigen Systems und der Blasenbildung kann ein Emulgator und/oder Tensid in die Mischung eingearbeitet werden. Diese Stoffe haben rein physikalische Effekte und sind nicht immer notwendig, insbesondere dann, wenn dichtere Schaumstoffe erwünscht sind. Jedes beliebige unter den Hunderten von üblichen Tensiden kann in Anteilen von bis zu 4 Gew.-%, bezogen auf das eingesetzte Polyol, verwendet werden. Geeignete Tenside sind das Polydimethylsiloxan und Polydimethylsiloxanpolyalkylencopolymere und ähnliche Verbindungen des Standes der Technik.

Es liegt auch im Rahmen der vorliegenden Erfindung, dort andere Stoffe in den Zusammensetzungen einzusetzen, wo ein besonderes Endresultat erwünscht ist. Als Stoffe dieser Art kommen, ohne dass dies als Beschränkung verstanden wird, Haftvermittler, Antioxydantien, Antistatika, antimikrobielle Mittel, Färbemittel, Hitze- und Lichtstabilisatoren, Pigmente, Weichmacher, Konservierungsmittel, UV-Stabilisatoren und Füllstoffe gemäss Beschreibung in "Modern Plastics Encyclopedia", Bd. 58, Nr. 10A, S. 170 - 187 in Betracht.

Die Erfindung wird durch die nachstehenden Beispiele, worin "Teile" Gewichtsteile bedeuten, erläutert.

**Beispiel 1**

Man erhitzt unter Rühren 49,6 Teile Dimethylmethylphosphonat und 245,6 Teile einer mit Propylenoxyd umgesetzten p-Nonylphenol/Formaldehyd/Diäthanolamin-Mannichbase (erhältlich unter dem Handelsnamen Thanol R650x®) auf 120°C unter Stickstoffatmosphäre. Das Rühren wird 4 Stunden lang bei 120°C fortgesetzt. Anschliessend verbringt man das Reaktionsgemisch in einen Rotationsverdampfer und entfernt die flüchtigen Stoffe durch Erhitzen auf 90°C für 2 Stunden unter einem Druck von 133 Pa. Der Rückstand besteht aus 266 Teilen einer viskosen Flüssigkeit. Die chemische $^{31}P$ NMR-Verschiebung des Produkts beträgt 23,8 ppm (feldabwärts = df - downfield), das Kennzeichen eines Amin/Phosphonsäuresalzes.

**Beispiel 2**

Man lässt in ähnlicher Weise wie in Beispiel 1 beschrieben, 81,8 Teile Dimethylmethylphosphonat und 202,6 Teile Thanol R650x® miteinander reagieren und erhält 256 Teile eines viskosen flüssigen Produkts; chemische Verschiebung $^{31}P$ = 23,5 (df).

**Beispiel 3**

In ähnlicher Verfahrensweise wie in Beispiel 1 angegeben, lässt man 24,8 Teile Dimethylmethylphosphonat und 106,0 Teile einer mit Propylenoxyd umgesetzten Phenol/Formaldehyd/Diäthanolamin/Mannichbase (erhältlich unter dem Handelsnamen Thanol R350x®) miteinander reagieren und erhält 128,5 Teile eines flüssigen, viskosen Produkts; chemische Verschiebung $^{31}P$ = 24,2 (df).

**Beispiel 4**

In ähnlicher Verfahrensweise wie in Beispiel 1 angegeben, lässt man 49,6 Teile Dimethylmethylphosphonat und 106,0 Teile Thanol R350x® miteinander reagieren und erhält 155,2 Teile eines flüssigen, viskosen Produkts; chemische $^{31}P$-Verschiebung = 24,4 (df).

**Beispiel 5**

In ähnlicher Verfahrensweise wie in Beispiel 1 beschrieben, lässt man 62,0 Teile Dimethylmethylphosphonat und 74,5 Teile Triäthanolamin exotherm miteinander reagieren und erhält 132,2 Teile eines flüssigen, viskosen Produkts; chemische $^{31}P$-Verschiebung = 21,4 (df).

Die Elementaranalyse des Produkts ergibt:
C = 38,8 %, H = 9,7 %, N = 5,2 %, P = 11,1 %.
Die theoretischen Werte von $C_9H_{24}NO_6P$ sind:
C = 39,6 %, H = 8,8 %, N = 5,1 %, P = 11,4 %.
Bei dem Produkt handelt es sich im wesentlichen um ein Amin/Phosphonatsalz.

**Beispiel 6**

In ähnlicher Verfahrensweise wie in Beispiel 1 beschrieben, lässt man 12,4 Teile Dimethylmethylphosphonat und 10,5 Teile Diäthanolamin exotherm miteinander reagieren und erhält 22,3 Teile eines flüssigen, viskosen Produkts; die chemische $^{31}P$-Verschiebung beträgt 22,0 (df).

Die Elementaranalyse des Produkts ergibt:
C = 36,0 %, H = 9,4 %, N = 6,0 %, P = 13,3 %.
Die theoretischen Werte von $C_7H_{20}NO_5P$ sind:
C = 36,7 %, H = 8,7 %, N = 6,1 %, P = 13,5 %.
Bei dem Produkt handelt es sich im wesentlichen um das Amin/Phosphonatsalz.

**Beispiel 7**

In ähnlicher Verfahrensweise wie in Beispiel 1 angegeben, lässt man 31,0 Teile Dimethylmethylphosphonat und 67,5 Teile eines Äthylendiamin/Alkylenoxydkondensats (erhältlich unter dem Handelsnamen Propylan A260®) miteinander reagieren und erhält 96,1 Teile eines flüssigen, viskosen Produkts; chemische $^{31}P$-Verschiebung = 21,3 (df).

**Beispiel 8**

Unter Rühren erhitzt man 11,0 Teile Methylphosphonsäuremonomethylester und 61,4 Teile Thanol R60x® auf 80°C. Man setzt das Rühren eine halbe Stunde bei 80°C fort und verbringt anschliessend das Reaktionsgemisch in einen Rotationsverdampfer, worin alle flüchtigen Stoffe unter 1-stündigem Erhitzen auf 80°C bei einem Druck von 133 Pa entfernt werden. Der Rückstand besteht aus 68 Teilen einer viskosen Flüssigkeit; die chemische $^{31}P$-Verschiebung beträgt 24,9 (df).

**Beispiel 9**

In ähnlicher Verfahrensweise wie in Beispiel 8 beschrieben, lässt man 22,0 Teile Methylphosphonsäuremonomethylester mit 61,4 Teilen Thanol R650x® reagieren und erhält 80,2 Teile eines flüssigen, viskosen Produkts; die chemische $^{31}P$-Verschiebung beträgt 25,2 (df).

**Beispiel 10**

In ähnlicher Verfahrensweise wie in Beispiel 8 beschrieben, lässt man 22,0 Teile Methylphosphonsäuremonomethylester mit 29,8 Teilen Triäthanolamin reagieren und erhält 51,0 Teile eines flüssigen, viskosen Produkts; die chemische $^{31}P$-Verschiebung = 19,6 (df).

**Beispiel 11**

Man erhitzt 19,2 Teile Methylphosphonsäure und 122,8 Teile Thanol R60x® miteinander unter Rühren auf 100°C. Man setzt das Rühren eine halbe Stunde bei 100°C fort. Danach wird das Reaktionsgemisch in einen Rotationsverdampfer verbracht und alle flüchtigen Stoffe durch 1-stündiges Erhitzen bei 80°C unter einem Druck von 133 Pa entfernt. Der Rückstand besteht aus 141,6 Teilen einer hochviskosen Flüssigkeit; chemische $^{31}P$-Verschiebung = 22,0 (df).

**Beispiel 12**

In ähnlicher Verfahrensweise wie in Beispiel 11 beschrieben, lässt man 19,2 Teile Methylphosphonsäure mit 59,6 Teilen Triäthanolamin reagieren und erhält 76,7 Teile eines flüssigen, viskosen Produkts; chemische $^{31}P$-Verschiebung = 20,5 (df).

**Beispiel 13**

Man vermischt 25 g (0,22 Mol) 1,4-Diazabicyclo-[2.2.2]octan und 70 g (0,56 Mol) Dimethylmethylphosphonat und erhitzt auf 120°C 3 Stunden lang unter einer Stickstoffatmosphäre. Nach dem Abkühlen auf Raumtemperatur baut man die Apparatur für die Vakuumdestillation um und entfernt den Überschuss an Dimethylmethylphosphonat bei einem Druck von 1596 Pa durch Erhitzen auf 95°C. Man erhält 77,2 g (97,4 %) einer strohfarbenen Flüssigkeit, welche den Hauptpeak bei 20,9 (feldabwärts) mit $H_3PO_4$ als Referenz bei der $^{31}P$ NMR-Spektroskopie zeigt.

**Beispiel 14**

Man erhitzt 47 g (0,5 Mol) 2-Aminopyridin auf 140°C und tropft 124 g (1 Mol) Dimethylmethylphosphonat über einen Zeitraum von 5 Stunden hinzu. Man steigert die Reaktionstemperatur auf 180°C und hält diese Temperatur 9 Stunden lang konstant. Das Reaktionsgemisch wird auf 50°C heruntergekühlt und die Apparatur für die Destillation umgebaut. Die bei der Reaktion nicht verbrauchten Ausgangsverbindungen werden bei einem Druck von 1596 Pa bis zu einer Gefässtemperatur von 120°C entfernt. Man kühlt das Reaktionsgemisch auf 30°C herunter und löst die entstandene dunkelbraune Flüssigkeit in 250 ml Chloroform. Beim Abkühlen auf 5°C setzen sich aus der Lösung 30 g einer weissen kristallinen Verbindung ab, welche die folgenden Analysenwerte zeigt:

C 44,1 %, H 6,09 %, N 12,84 % und P 13,95 %

Berechnet für $C_8H_{15}N_2O_3P$:

C 44,0 %, H 6,88 %, N 12,84 % und P 14,22 %.

Die Verbindung schmilzt bei 220°C unter Zersetzung.

Die $^1H$ NMR- und $^{31}P$ NMR-Spektroskopiewerte weisen aus, dass es sich bei der Verbindung um

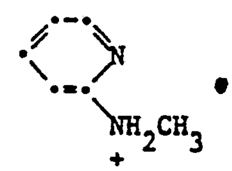
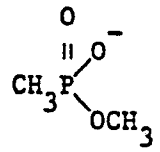

handelt.

**Beispiel 15**

Man evaporiert das nach Beispiel 15 erhältliche Chloroformfiltrat 3 Stunden lang bei 60°C und erhält so eine dunkelbraune Flüssigkeit, welche folgende Analysendaten aufweist:
C 38,01 %, H 7,21 %, N 8,34 % und P 17,97 %.
Berechnet für $C_{11}H_{24}N_2O_6P_2$:
C 38,60 %, H 7,02 %, N 8,18 % und P 18,13 %.
Die $^1H$ NMR- und $^{31}P$ NMR-Spektroskopiewerte zeigen, dass es sich bei dem Stoff um

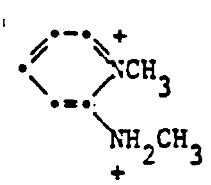
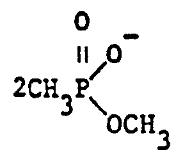

handelt.

**Beispiel 16**

Man löst 50,5 g (0,5 Mol) Triäthylamin und 62 g (0,5 Mol) Dimethylmethylphosphonat in 250 ml Mesitylen und erhitzt die Mischung unter Rückfluss insgesamt 22,5 Stunden lang, wobei die Rückflusstemperatur von 133°C auf 154°C zunimmt. Das Reaktionsprodukt scheidet sich aus der Lösung als braungefärbte Flüssigkeit ab. Man kühlt das Reaktionsgemisch auf 30°C ab und baut die Apparatur für die Destillation um. Man entfernt das Mesitylen und die bei der Reaktion nicht verbrauchten Ausgangsstoffe im Vakuum zuerst bei einem Druck von 1596 Pa und dann bei 133 Pa bei einer Temperatur bis zu 190°C. Danach wird die erhaltene dunkel gefärbte Flüssigkeit in Methanol gelöst und die so erhältliche Lösung durch Behandeln mit Aktivkohle entfärbt. Man erhält nach dem Abdampfen des Methanols 87 g (77,3 %) einer strohfarbenen Flüssigkeit, welche die folgenden Analysenwerte zeigt:
C 47,69 %, H 11,05 %, N 6,49 % und P 13,96 %.
Berechnet für $C_9H_{24}NO_3P$:
C 48,0 %, H 10,67 %, N 6,22 %, P 13,77 %.
Das Reaktionsprodukt zeigt einen einzigen Peak bei 21,1 (feldabwärts) mit $H_3PO_4$ als Referenz bei der $^{31}P$ NMR-Spektroskopie.

**Beispiel 17**

Man erhitzt 124 g (1 Mol) Dimethylmethylphosphonat auf 140°C unter einer Stickstoffatmosphäre und tropft während eines Zeitraums von 6 Stunden 30 g (0,5 Mol) 1,2-Diaminoäthan hinzu, wobei man eine Temperatur von 140°C aufrechterhält. Nach dem Abkühlen auf Raumtemperatur erhält man eine dunkelbraune harzähnliche Substanz. Die Entfärbung dieser Substanz erfolgt durch Auflösen in 300 ml Methanol und nachfolgendem Behandeln mit Aktivkohle. Beim Einengen der Lösung auf 200 ml und Abkühlen im Eisbad setzt sich ein schwachgefärbtes kristallines Material ab, welches sich durch Filtrieren abtrennen und im Vakuum trocknen lässt. Man erhält 67 g eines beinahe weissen Feststoffs mit einem Schmelzpunkt von 108 - 112°C. Die Analysenwerte dieser Substanz sind wie folgt:
C 30,03 %, H 8,66 %, N 9,39 %, P 20,0 %
Berechnet für $C_8H_{26}N_2O_6P_2$:

C 31,16 %, H 8,44 %, N 9,09 %, P 20,13 %.
Die Substanz zeigt einen einzigen Peak bei 28,6 (feldabwärts) mit $H_3PO_4$ als Referenz bei der $^{31}P$-Spektroskopie.

**Beispiel 18**

Man erhitzt 124 g (1 Mol) Dimethylmethylphosphonat bei 140°C unter einer Stickstoffatmosphäre, während 34,3 g (0,33 Mol) Diäthyltriamin innerhalb eines Zeitraums von 5 Stunden hinzugetropft werden. Danach kühlt man das Reaktionsgemisch auf 30°C ab und baut die Apparatur für die Vakuumdestillation um. Es werden die nicht verbrauchten Reaktanten bei einem Druck von 1596 Pa bei einer Gefässtemperatur von bis zu 150°C entfernt. Man erhält 155 g (97,9 % der Theorie) einer dunkelbraunen harzartigen Substanz. Die Elementaranalyse zeigt die folgenden Werte:
C 31,15 %, H 8,65 %, N 8,29 %, P 19,56 %.
Berechnet für $C_{13}H_{40}N_3O_9P_3$:
C 32,84 %, H 8,42 %, N 8,84 % und P 19,58 %.
Die $^{31}P$-Spektroskopie zeigt zwei Peaks; einen grösseren bei 22,6 und einen kleineren bei 21,4 (beide feldabwärts mit $H_3PO_4$ als Referenz).

**Beispiel 19**

Man erhitzt 62 g (0,5 Mol) Dimethylmethylphosphonat bei 140°C, während man innerhalb eines Zeitraums von 4 Stunden 39,5 g (0,5 Mol) Pyridin hinzutropft. Dann erhitzt man die Mischung für weitere 2 Stunden bei 140°C und kühlt sie danach auf 30°C ab. Man baut die Apparatur für die Destillation um und entfernt nicht verbrauchte Ausgangsstoffe bei einem Druck von 39,9 Pa und einer Temperatur von maximal 100°C. Man entfernt Spuren von Pyridin durch Auflösen der dunklen Reaktionsmasse in 200 ml Wasser und Behandeln der so erhältlichen Lösung mit Aktivkohle. Nach dem Evaporieren der Lösung bis zur Gewichtskonstanz erhält man 86,2 g (84,9 % der Theorie) eines dunkelbraunen Harzes. Die Analysendaten sind wie folgt:
C 47,98 %, N 7,09 %, N 6,09 % und P 16,07 %
Berechnet für ...:
C 47,29 %, H 6,89 %, N 6,89 % und P 15,27 %
Die $^{31}P$ NMR-Spektroskopie zeigt einen einzelnen Peak bei 33,4 (feldabwärts) mit $H_3PO_4$ als Referenz.

**Beispiel 20**

Man erhitzt 34 g (0,5 Mol) Imidazol bei 140°C und fügt während eines Zeitraums von 4 Stunden 124 g (1 Mol) Dimethylmethylphosphonat hinzu. Man lässt die Reaktionstemperatur innerhalb von 5 Stunden allmählich auf 185°C ansteigen und erhitzt bei dieser Temperatur weitere 6 Stunden lang. Man kühlt das Reaktionsgemisch auf 30°C ab und baut die Apparatur für die Vakuumdestillation um. Die unverbrauchten Ausgangsstoffe werden durch Destillieren bei einem Druck von 39,9 Pa und einer Gefässtemperatur von bis zu 110°C entfernt. Man erhält 122,5 g (77,5 % der Theorie) eines dunkelbraunen Harzes, welches die folgenden Analysenwerte zeigt:
C 34,81 %, H 7,43 %, N 9,11 %, P 19,49 %.
Berechnet für $C_9H_{22}N_2O_6P_2$:
C 34,17 %, H 6,96 %, N 8,86 %, P 19,62 %.

**Beispiel 21**

Man erhitzt 43 g (0,5 Mol) Piperazin bei 140°C und tropft während eines Zeitraums von 5 Stunden 124 g (1 Mol) Dimethylmethylphosphonat hinzu und erhitzt die Reaktionsmasse anschliessend bei 150°C 4,5 Stunden lang. Nach dem Abkühlen auf 30°C löst man das bernsteinfarbene Harz in 125 ml Äthanol auf. Man kühlt die so erhaltene Lösung auf -70°C ab und isoliert 53,1 g einer weissen kristallinen Verbindung durch Filtrieren. Diese Verbindung hat einen Schmelzpunkt von 251 - 253°C unter Zersetzung und weist die folgenden Elementaranalysenwerte auf:
C 34,96 %, H 8,77 %, N 8,51 %, P 19,17 %
Berechnet für $C_{10}H_{28}N_2O_6P_2$:
C 35,92 %, H 8,38 %, N 8,38 %, P 18,56 %.
Die $^{31}P$ NMR-Spektroskopie zeigt einen einzigen Peak bei 24,6 (feldabwärts) mit $H_3PO_4$ als Referenz.

**Beispiel 22**

Man erhitzt 62 g (0,5 Mol) Dimethylmethylphosphonat bei 140°C und fügt 42,5 g (0,5 Mol) Piperidin während eines Zeitraums von 5 Stunden hinzu. Man setzt das Erhitzen bei 140°C weitere 7 Stunden lang fort. Nach dem Abkühlen auf 30°C rüstet man die Apparatur für die Destillation um und entfernt die nicht verbrauchten Ausgangsstoffe bei einem Druck von 53,2 Pa bei einer Gefässtemperatur von bis zu 150°C. Man erhält 84,5 g (80,9 % der Theorie) einer braunen viskosen Flüssigkeit mit folgenden Elementaranalysenwerten:
C 45,38 %, H 9,50 %, N 7,05 %, P 15,16 %
Berechnet für $C_8H_{20}NO_3P$:
C 45,93 %, H 9,57 %, N 6,70 %, P 14,83 %.

**Beispiel 23**

Man erhitzt 62 g (0,5 Mol) Dimethylmethylphosphonat bei 140°C und tropft während eines Zeitraums von 4 Stunden 43,5 g (0,5 Mol) Morpholin hinzu. Das Reaktionsgemisch beginnt unter Rückfluss zu kochen, und die Temperatur fällt auf 134°C ab. Man hält das Reaktionsgemisch weitere 3 Stunden lang bei dieser Temperatur. Nach Entfernung der nicht verbrauchten Ausgangsstoffe durch Vakuumdestillation bei einem Druck von 39,9 Pa und einer Gefässtemperatur von bis zu 120°C erhält man 70 g einer dunklen, viskosen Flüssigkeit. Die Elementaranalyse ergibt folgende Werte:
C 39,47 %, H 8,43 %, N 6,22 %, P 14,8 %.
Berechnet für $C_7H_{18}NO_4P$:
C 39,81 %, H 8,53 %, N 6,63 %, P 14,70 %.

**Beispiel 24**

Man fügt 30 g (0,5 Mol) 1,2-Diaminoäthan innerhalb von 1 Stunde zu einer Lösung von 48 g (0,5 Mol) Methylphosphonsäure in 200 ml Methanol. Man hält das Gemisch auf einer Temperatur von <25°C durch Kühlung in einem Eiswasserbad. Nach 1-stündigem fortgesetztem Rühren verdünnt man das Reaktionsgemisch mit 50 ml Methanol und trennt das Reaktionsprodukt durch Filtrieren ab. Nach dem Vakuumtrocknen bei 50°C erhält man 75,1 g (96,3 % der Theorie) einer weissen kristallinen Substanz mit einem Schmelzpunkt von 242 - 244°C und den folgenden Analysenwerten:
C 22,79 %, H 8,2 %, N 17,46 %, P 19,93 %.
Berechnet für $C_3H_{13}N_2O_3P$:
C 23,08 %, H 8,39 %, N 17,9 %, P 19,84 %.

**Beispiel 25**

Während eines Zeitraums von 1 Stunde fügt man 107 g (1 Mol) Benzylamin zu 124 g (1 Mol) Dimethylmethylphosphonat bei 140°C unter einer Stickstoffatmosphäre hinzu. Man erwärmt das Reaktionsgemisch bei 140°C weitere 3 Stunden lang und kühlt es danach auf 60°C ab. Man rüstet die Apparatur für die Vakuumdestillation um und entfernt die nicht verbrauchten Ausgangsstoffe bei einem Druck von 1596 Pa und einer Gefässtemperatur von bis zu 140°C. So erhält man 220,5 g einer extrem hygroskopen gelb gefärbten Flössigkeit, welche einen Phosphorgehalt von 13,05 % aufweist.
Für $C_{10}H_{18}NO_3P$ wurde ein P-Gehalt von 13,39 % berechnet.

**Beispiel 26**

Man fügt innerhalb von 1 Stunde 44 g (0,5 Mol) N,N'-Dimethyläthylendiamin zu 124 g (1 Mol) Dimethylmethylphosphonat bei 140°C unter Stickstoffatmosphäre hinzu. Während dieser Zugabe bewirkt eine exotherme Reaktion eine Steigerung der Temperatur auf 165°C. Das Reaktionsgemisch kühlt man auf 140°C ab und hält diese Temperatur weitere 3 Stunden lang aufrecht, kühlt danach auf 50°C ab und rüstet die Apparatur för die Vakuumdestillation um. Die nicht in die Reaktion eingegangenen Ausgangsstoffe werden durch Destillation bei einer Temperatur von bis zu 140°C bei einem Druck von 1596 Pa entfernt. Man erhält 153,8 g (91,5 %) einer braun gefärbten hygroskopen Flüssigkeit, welche die folgenden Analysenwerte zeigt:
C 35,17 %, H 8,58 %, N 8,65 %, P 18,44 %.
Berechnet für $C_{10}H_{30}N_2O_6P_2$:
C 35,71 %, H 8,93 %, N 8,33 %, P 18,45 %

**Beispiel 27**

Man fügt 75 g (1 Mol) 2-Methoxyäthylamin innerhalb 1 Stunde zu 124 g (1 Mol) Dimethylmethylphosphonat bei 140°C unter einer Stickstoffatmosphäre hinzu. Diese Temperatur wird für weitere 3 Stunden aufrechterhalten und danach wird auf 40°C abgekühlt. Man baut die Apparatur für die Vakuumdestillation um und entfernt die unverbrauchten Ausgangsmaterialien durch allmähliches Erhitzen auf eine Temperatur von maximal 140°C bei einem Druck von 1596 Pa. Man gewinnt so 158,8 g (79,8 % der Theorie) einer bernsteinfarbenen Flüssigkeit, welche die folgenden Analysenwerte zeigt:
C 35,95 %, H 9,15 %, N 7,31 %, P 15,89 %.
Berechnet für $C_6H_{18}NO_4P$:
C 36,17 %, H 9,12 %, N 7,03 %, P 15,54 %.

**Beispiel 28**

Man fügt 64,2 g (0,6 Mol) N-Methylanilin innerhalb von 1 Stunde tropfenweise zu einer Lösung von 66 g (0,6 Mol) Monomethylmethylphosphonat in 100 ml Methanol, welche in einem Eis/Wasserbad bei <15°C gekühlt wird. Die so erhältliche Lösung wird noch 1 Stunde lang weitergerührt, wonach das Methanol durch Vakuumdestillation entfernt wird. Man erhält 130,1 g einer dunkelbraunen Flüssigkeit mit den folgenden Analysenwerten:
C 49,74 %, H 7,46 %, N 6,34 %, P 14,60 %
Berechnet für $C_9H_{16}NO_3P$:
C 49,77 %, H 7,37 %, N 6,45 %, P 14,29 %
Das Produkt zeigt einen einzigen Peak bei 27,2 (feldabwärts) mit $H_3PO_4$ als Referenz bei der $^{31}P$ NMR-Spektroskopie.

**Beispiel 29**

Man fügt 72,6 g (0,6 Mol) N,N-Dimethylanilin innerhalb von 1 Stunde zu einer Lösung von 66 g (0,6 Mol) Monomethylmethylphosphonat in 100 ml Methanol, während man bei <15°C mittels eines Eis/Wasserbads kühlt. Die so erhältliche Lösung wird noch 1 Stunde lang weitergerührt und danach das Methanol durch Vakuumdestillation entfernt. Man erhält 138,1 g einer blassbräunlichen Flüssigkeit mit den folgen Analysendaten:
C 51,67 %, H 8,00 %, N 5,80 %, P 13,65 %.
Berechnet für $C_{10}H_{18}NO_3P$:
C 51,94 %, H 7,79 %, N 6,06 %, P 13,42 %
Das Produkt zeigt einen einzigen Peak bei 28,0 (feldabwärts) mit $H_3PO_4$ als Referenz bei der $^{31}P$ NMR-Spektroskopie.

**Beispiel 30**

Man erhitzt 55,5 g (0,5 Mol) Chinuclidin bei 120°C unter einer Stickstoffatmosphäre und fügt 62 g (0,5 Mol) Dimethylmethylphosphonat innerhalb von 1 Stunde tropfenweise hinzu. Man hält das Reaktionsgemisch für weitere 4 Stunden bei 120°C und kühlt es dann auf 40°C ab, löst es in 200 ml Methanol und behandelt es mit Aktivkohle. Man erhält nach der Filtration und Vakuumtrocknung 115,1 g (97,9 % der Theorie) einer blassgelblichen hygroskopen Flüssigkeit. Das Produkt zeigt die folgenden Analysenwerte:
C 46,29 %, H 9,52 %, N 5,26 %, P 11,56 %.
Berechnet für $C_{10}H_{22}NO_3P \cdot 1{,}5H_2O$:
C 48,80 %, H 9,54 %, N 5,34 %, P 11,83 %.

**Beispiel 31**

Man erhitzt 44 g (0,5 Mol) N,N-Dimethyläthylendiamin unter einer Stickstoffatmosphäre auf 140°C und tropft innerhalb von 1 Stunde 124 g (1 Mol) Dimethylmethylphosphonat hinzu. Man hält die Temperatur des Reaktionsgemischs für weitere Stunden auf 140°C. Nach dem Abkühlen auf 40°C rüstet man die Apparatur für die Destillation um und entfernt die nicht verbrauchten Ausgangsstoffe bei einem Druck von 1596 Pa und bei einer Temperatur von bis zu 140°C. Man erhält 149,6 g (89,0 % der Theorie) einer braunen Flüssigkeit, welche beim Stehenlassen teilweise auskristallisiert. Das Produkt zeigt die folgenden Analysenwerte:

C 35,39 %, H 8,61 %, N 8,43 %, P 18,41 %.
Berechnet für $C_{10}H_{30}N_2O_6P_2$:
C 35,71 %, H 8,93 %, N 8,33 %, P 18,45 %.

**Beispiel 32**

Man fügt 148,5 g (1,5 Mol) Cyclohexylamin unter einer Stickstoffatmosphäre innerhalb von 2 Stunden zu 186 g (1,5 Mol)-Dimethylmethylphosphonat bei 140°C. Man hält das Reaktionsgemisch weitere 2 Stunden lang bei 140°C und kühlt danach auf 70°C ab. Man baut die Apparatur für die Destillation um und entfernt die nicht verbrauchten Ausgangsstoffe durch Destillation bei einem Druck von 1596 Pa und einer Temperatur von bis zu 140°C. Man erhält 317,1 g (94,8 % der Theorie) eines blassgelblichen, viskosen harzähnlichen Stoffs mit den folgenden Analysendaten:
C 48,46 %, H 9,95 %, N 5,98 %, P 13,67 %.
Berechnet für $C_9H_{22}NO_3P$:
C 48,43 %, H 9,87 %, N 6,28 %, P 13,90 %.
Die $^{31}P$ NMR-Spektroskopie zeigt einen einzigen Peak bei 22,9 (feldabwärts) mit $H_3PO_4$ als Referenz.

**Beispiel 33**

Man fügt 46,5 g (0,5 Mol) Anilin innerhalb von 30 Min. zu einer Lösung von 48 g (0,5 Mol) Methylphosphonsäure in 200 ml Methanol bei <15°C. Die aus der Lösung abgesetzte weisse kristalline Verbindung trennt man durch Filtration ab und trocknet sie im Vakuum. Man erhält 76,7 g einer Substanz mit einem Schmelzpunkt von 144 - 146°C, welche die folgenden Analysendaten hat:
C 44,33 %, H 6,37 %, N 7,36 %, P 16,60 %.
Berechnet für $C_7H_{12}NO_3P$:
C 44,44 %, H 6,35 %, N 7,41 %, P 16,40 %.

**Beispiele 34 - 70**

Die folgenden Beispiele illustrieren die Leichtigkeit, mit welcher erfindungsgemäss flammhemmende Polyurethan-Hartschaumstoffmassen aus Polyolen und polymerem Diphenylmethandiisocyanat herstellbar sind.

Die folgende Schaumstofformulierung dient zur Erläuterung der flammhemmenden Wirkung.

| **Reaktionspartner** | **Konzentration** (Teile) |
|---|---|
| Thanol R650x®1 | wie angegeben |
| Wasser | 0,2 |
| Silikontensid | 2 |
| Trichlorfluormethan | 40 zur Erzielung einer Schaumstoffdichte von 30 ± 1 kg/cm³) |
| Flammhemmendes Mittel | wie angegeben |
| Suprasec DND®2 | bis zu einem Index von 1,05 |

1. Ein aromatisches Polyol
2. Ein polymeres Diphenylmethandiisocyanat.

Man vermischt die oben bezeichneten Bestandteile 10 Sekunden lang mit Hilfe eines Hochgeschwindigkeitsrührers (2000 Upm) bei Raumtemperatur miteinander, wobei das Isocyanat zuletzt hinzugefügt und die Mischung unmittelbar darauf in eine Kartonform gegossen wird. Die eintretende exotherme Reaktion lässt den Schaum frei aufsteigen. Die Zeitdauer ab Isocyanatzugabe bis zur Bildung einer rahmigen Konsistenz des Gemischs wird als Rahmzeit angegeben. Die für das Erreichen der maximalen Schaumhöhe benötigte Zeit wird als Steigzeit angegeben. Die Zeit, bis der Schaum seine Klebrigkeit verliert, wird als Berührungstrockenzeit angegeben. Nach Erreichen der Berührungstrockenzeit wird der Schaumstoff 3 Tage lang bei Raumtemperatur gealtert.

Nach 3-tägiger Lagerungszeit schneidet man Testmuster aus dem Schaumstoff und unterwirft sie der Grenzsauerstoffkonzentrationsprobe und der Senkrecht-Brennprobe nach DIN 4102 B2. Die Ergebnisse sind in der untenstehenden Tabelle dargestellt, wobei zum Vergleich der gleiche Schaumstoff, jedoch ohne flammhemmendes Mittel, hergestellt wurde (Beispiel 34). Die nach den Beispielen 35 - 70 hergestellten Schaumstoffe zeigten weder Risse, noch Verwerfungen oder Versengungen.

| Bsp. | Produkt gem. Bsp. Nummer | Menge (Teile) | Polyol Teile | Schaumstoffparameter Rahmzeit (Sek.) | Steigzeit (Sek.) | Berührungstrockenzeit (Sek.) | Sauerstoffindex % |
|---|---|---|---|---|---|---|---|
| 34 | None | None | 100 | 17 | 70 | 120 | <21 |
| 35 | 1 | 65 | 45 | 12 | 18 | 25 | 23,8 |
| 36 | 2 | 38 | 72 | 10 | 18 | 24 | 24,7 |
| 37 | 3 | 52,7 | 57,3 | 14 | 22 | 34 | 24,3 |
| 38 | 4 | 31,3 | 78,7 | 15 | 30 | 40 | 24,3 |
| 39 | 5 | 19,7 | 90 | 19 | 25 | 35 | 24,5 |
| 40 | 6 | 18,3 | 91,7 | 10 | 23 | 39 | 22,4 |
| 41 | 7 | 31 | 80 | 16 | 20 | 34 | 23,9 |
| 42 | 8 | 52,4 | 57,6 | 21 | 52 | 81 | 23,8 |
| 43 | 9 | 34,1 | 74,8 | 12 | 20 | 30 | 24,2 |
| 44 | 10 | 25,7 | 100 | 10 | 22 | 34 | 24,6 |
| 45 | 11 | 58 | 49,7 | 10 | 15 | 23 | 24,1 |
| 46 | 12 | 41,4 | 100 | 11 | 29 | 51 | 24,6 |
| 47 | 13 | 24,7 | 100 | 11 | 24 | 43 | 22,8 |
| 48 | 14 | 10 | 100 | 9 | 21 | 35 | 22,7 |
| 49 | 14 | 16 | 100 | 8 | 20 | 37 | 23,5 |
| 50 | 15 | 10 | 100 | 21 | 75 | 140 | 23,6 |
| 51 | 16 | 10 | 100 | 10 | 20 | 34 | 25,0 |
| 52 | 17 | 10 | 100 | 10 | 14 | 19 | 24,2 |
| 53 | 18 | 13 | 100 | 12 | 24 | 30 | 24,3 |
| 54 | 19 | 13 | 100 | 7 | 15 | 29 | 24,4 |
| 55 | 20 | 10 | 100 | 11 | 16 | 26 | 24,6 |
| 56 | 21 | 10 | 100 | 8 | 15 | 27 | 24,8 |
| 57 | 22 | 10 | 100 | 21 | 79 | 134 | 25,0 |
| 58 | 23 | 10 | 100 | 9 | 16 | 28 | 25,0 |
| 59 | 24 | 10 | 100 | 9 | 19 | 31 | 25,1 |
| 60 | 25 | 10 | 100 | 22 | 75 | 138 | 22,9 |
| 61 | 26 | 10 | 100 | 4 | 10 | 20 | 24,7 |
| 62 | 27 | 10 | 100 | 10 | 19 | 34 | 25,4 |
| 63 | 28 | 10 | 100 | 9 | 19 | 32 | 25,7 |
| 64 | 29 | 10 | 100 | 9 | 26 | 56 | 24,3 |
| 65 | 30 | 10 | 100 | 15 | 34 | 77 | 24,1 |
| 66 | 31 | 10 | 100 | 4 | 11 | 28 | 24,8 |
| 67 | 32 | 10 | 100 | 5 | 16 | 28 | 25,2 |
| 68 | 33 | 10 | 100 | 5 | 17 | 30 | 23,7 |
| 69 | 34 | 10 | 100 | 8 | 34 | 70 | 24,9 |
| 70 | 2 | 19 | 86 | 13 | 27 | 37 | 25,2 |
| | 18 | 5 | | | | | |

(Fortsetzung)

| Bsp. | DIN 4102 B2 Test Zeit bis zum Spezifizierungszeichen (Sek.) | Maximale Flammenhöhe (cm) | Brenndauer (Sek.) |
|---|---|---|---|
| 34 | 3 | >20 | >60 verbrennt vollständig |
| 35 | - | 12 | 17 |
| 36 | - | 14 | 17 |
| 37 | - | 14 | 12 |
| 38 | - | 13 | 10 |
| 39 | - | 13 | 13 |
| 40 | - | 14 | 10 |
| 41 | 7 | 15 | 14 |
| 42 | - | 12 | 8 |
| 43 | 5 | 15 | 15 |
| 44 | - | 14 | 15 |

| | | | |
|---|---|---|---|
| 45 | - | 14 | 14 |
| 46 | 10 | 15 | 14 |
| 47 | 10 | 15 | 15 |
| 48 | - | 13 | 10 |
| 49 | - | 13 | 9 |
| 50 | - | 13 | 10 |
| 51 | - | 12 | 12 |
| 52 | - | 16 | 13 |
| 53 | - | 12 | 9 |
| 54 | - | 14 | 11 |
| 55 | - | 13 | 10 |
| 56 | - | 12 | 15 |
| 57 | - | 12 | 7 |
| 58 | - | 14 | 15 |
| 59 | - | 12 | 11 |
| 60 | - | 14 | 10 |
| 61 | - | 11 | 14 |
| 62 | - | 12 | 12 |
| 63 | - | 13 | 11 |
| 64 | 6 | 15 | 12 |
| 65 | 6 | 17 | 14 |
| 66 | 7 | 15 | 10 |
| 67 | 8 | 15 | 12 |
| 68 | 4 | 17 | 12 |
| 69 | 8 | 15 | 12 |
| 70 | - | 12 | 8 |

**Beispiele 71 - 74**

Die folgenden Beispiele illustrieren die Leichtigkeit, mit welcher erfindungsgemäss flammhemmende elastische Polyurethanschaumstoffmassen aus Polyolen und Toluoldiisocyanat herstellbar sind.

Die folgende Schaumstofformulierung dient zur Erläuterung der flammhemmenden Wirkung.

| **Reaktionspartner** | **Konzentration** (Teile) |
|---|---|
| Propylan B383®[1] | 100 |
| Silikontensid | 1 |
| Wasser | 4 (zur Erzielung einer Schaumstoffdichte von $28+1$ $kg/cm^3$) |
| Zinn(II)octoat | 0,3 |
| Propamine A®[2] | 0,4 |
| Flammhemmendes Mittel | wie angegeben |
| Toluoldiisocyanat | bis zu einem Index von 1,08 |

Die oben genannten Bestandteile werden mit Hilfe eines Handschnellrührgeräts (2000 Upm) 10 Sekunden lang bei Raumtemperatur miteinander vermischt und dann so schnell wie möglich in eine Kartonform gegossen. Die eintretende exotherme Reaktion gestattet das freie Aufsteigen des Schaums. Nach 7-tägigem Aushärten des Schaums bei Raumtemperatur wurden die in der nachstehenden Tabelle dargestellten physikalischen Eigenschaften festgestellt.

Zum Vergleich wurde ein Schaumstoff aus den gleichen Reaktionspartnern ohne Zusatz eines flammhemmenden Mittels hergestellt, wobei ebenfalls ein Isocyanatindex von 1,08 eingehalten wurde.

Nach 3-tägiger Lagerungszeit wurden aus dem Schaumstoff Testmuster herausgeschnitten und der Horizontal, Brennprobe BS 4735 unterzogen. Die Ergebnisse sind in der nachstehenden Tabelle dargestellt.

| | Produkt nach Beispiel | | BS 4735 Brenntest | |
|---|---|---|---|---|
| Beispiel Nr. | Nummer | Menge (Teile) | Brennlänge (mm) | Brenndauer (Sek.) |
| - | keine | nichts | verbrennt | vollständig |
| 71 | 4 | 22,6 | 61 | 52 |
| 72 | 5 | 13,7 | 90 | 65 |
| 73 | 14 | 10 | 28 | 33 |
| 74 | 18 | 13,7 | 90 | 65 |

**Beispiele 75 - 76**

Es wird im folgenden die Lagerstabilität einer erfindungsgemässe Produkte enthaltenden Polyolformulierung illustriert. Es wurden zwei Formulierungen hergestellt und deren Viskosität nach der Herstellung und nach 39-tägiger Lagerung gemessen. In einem Fall war nur eine geringfügige Differenz und im anderen Fall keine Änderung in der Viskosität festzustellen.

| **Formulierung** | Beispiel 75 | Beispiel 76 |
|---|---|---|
| Produkt gemäss Beispiel 1 | 65 Teile | 0 |
| Produkt gemäss Beispiel 2 | 0 | 38 |
| Thanol R650x® | 45 | 72 |
| Silicone DC 193® | 2 | 2 |
| Refrigerant 11 | 40 | 40 |
| Isopropyliertes Phenylphosphat | 15 | 15 |
| **Eigenschaften** | | |
| Viskosität $mm^2/S$ am Tag 0 | 1463 | 780 |
| Viskosität $mm^2/S$ am Tag 39 | 1446 | 780 |

**Patentansprüche**

1. Aminsalze von Phosphonsäuren der allgemeinen Formel Ia und/oder Ib

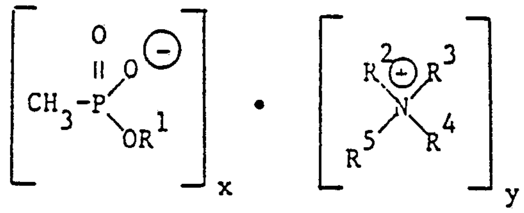

(Ia)

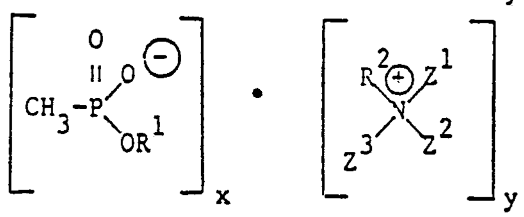

(Ib)

worin

x und y solche ganzen Zahlen sind, dass die Anzahl negativer und positiver Ladungen gleich ist und

$R^1$ Wasserstoff, Methyl oder eine negative Ladung bedeutet,

$R^2$ Wasserstoff oder Methyl,

$R^3$ eine durch 1 - 3 gegebenenfalls durch eine Oxyalkylenkette veretherte Hydroxylgruppen substituierte $C_2$-$C_4$-Alkylgruppe bedeuten, wobei sich nicht mehr als eine Hydroxylgruppe an jeweils einem Kohlenstoffatom befindet,

$R^4$ und $R^5$ gleich oder verschieden sind und dieselbe Bedeutung wie $R^3$ haben können oder Wasserstoff, eine $C_1$-$C_4$-Alkyl-, Phenyl-, Benzylgruppe oder eine am aromatischen Ring durch eine $C_1$-$C_{12}$-Alkylgruppe, eine gegebenenfalls durch eine Oxyalkylenkette veretherte Hydroxylgruppe und/oder 1 - 3 Halogenatome substituierte Phenyl- oder Benzylgruppe bedeuten

oder $R^5$ eine Gruppe der Formel II

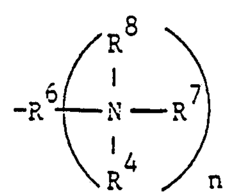

(II)

bedeutet, worin $R^6$ eine $C_2$-$C_4$-Alkylen-, Phenylen-, Xylylen- oder Diphenylmethangruppe oder eine Gruppe der Formel

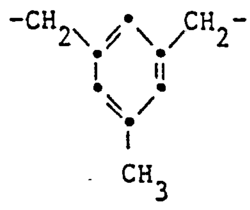

oder

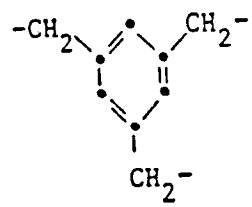

ist, wobei aromatische Ringe in einer $R^6$-Gruppe gegebenenfalls durch eine $C_1$-$C_{12}$-Alkylgruppe, eine gegebenenfalls durch eine Oxyalkylenkette veretherte Hydroxylgruppe und/oder 1 - 3 Halogenatome substituiert sein können, und

$R^7$ Wasserstoff oder Methyl bedeutet, wobei das Stickstoffatom eine positive Ladung trägt, oder $R^7$ nicht vorhanden ist;

$R^8$ Wasserstoff ist oder dieselbe Bedeutung wie $R^3$ hat und

n 1 oder 2 bedeutet

oder $R^4$ und $R^5$ gegebenenfalls zusammen mit dem Stickstoffatom einen gegebenenfalls ein Sauerstoffatom enthaltenden 5- oder 6-gliedrigen heterocyclischen Ring bilden; sowie

$Z^1$, $Z^2$ und $Z^3$ gleich oder verschieden sind und Wasserstoff, eine gerad- oder verzweigtkettige $C_1$-$C_{12}$-Alkyl- bzw. $C_3$-$C_{12}$-Alkenyl- oder Alkinylgruppe, eine $C_4$-$C_{12}$-Cycloalkylgruppe, eine gegebenenfalls durch eine gerad- oder verzweigtkettige $C_1$-$C_4$-Alkylgruppe, eine $C_1$-$C_4$-Alkoxygruppe, Amino, Methylamino, Halogen oder Nitro substituierte Phenyl- oder Naphthylgruppe oder eine $C_7$-$C_{12}$-Aralkylgruppe bedeuten

oder $Z^2$ und $Z^3$ zusammen mit dem sie verknüpfenden Stickstoffatom ein gesättigtes oder ungesättigtes 3- bis 7-gliedriges Ringsystem bilden, welches gegebenenfalls ein weiteres Heteroatom enthält und gegebenenfalls durch eine gerad- oder verzweigtkettige $C_1$-$C_4$-Alkylgruppe, eine $C_1$-$C_4$-Alkoxygruppe, Amino, Methylamino, eine $C_1$-$C_4$-Aminoalkylgruppe, Halogen oder Nitro substituiert sein kann,

oder $Z^1$, $Z^2$ und $Z^3$ zusammen mit dem sie verknüpfenden Stickstoffatom eine gegebenenfalls ein weiteres Heteroatom enthaltenden 8- bis 12-gliedrigen bicyclischen Ring bilden,

oder $Z^1$ eine Gruppe der Formel

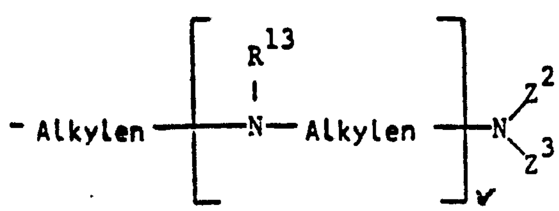

darstellt, worin

Alkylen eine Gruppe mit 2 - 12 Kohlenstoffatomen,

v 0 oder eine ganze Zahl von 1 - 5,

$R^{13}$ Wasserstoff oder eine gerad- bzw. verzweigtkettige $C_1$-$C_{16}$-Alkylgruppe bedeuten und

$Z^2$ sowie $Z^3$ dieselbe Bedeutung wie obenstehend definiert besitzen,

oder $Z^1$ eine Gruppe der Formel

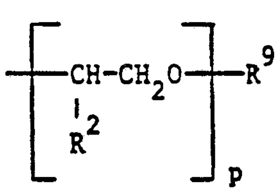

oder

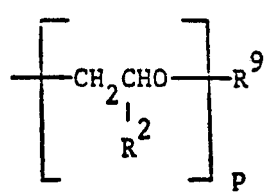

ist, worin

$R^2$ die oben angegebene Bedeutung hat und

$R^9$ eine $C_1$-$C_{12}$-Alkylgruppe und

p eine ganze Zahl von 1 bis 10, vorzugsweise 1 - 4 bedeuten;

oder $Z^3$ eine Gruppe der Formel IIa

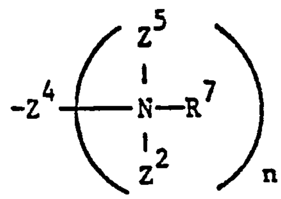

(IIa)

darstellt, worin $Z^4$ eine $C_2$-$C_{12}$-Alkylengruppe, eine Phenylen-, Xylylen- oder Diphenylmethangruppe oder eine Gruppe der Formel

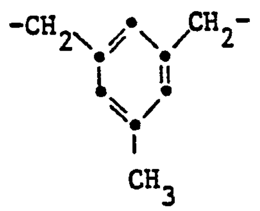

oder

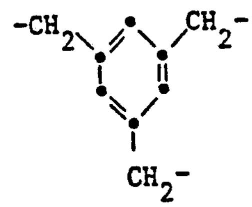

bedeutet, wobei aromatische Ringe in einer $Z^4$-Gruppe gegebenenfalls durch eine $C_1$-$C_{12}$-Alkylgruppe, Hydroxyl und/oder 1 - 3 Halogenatome substituiert sein können, und

$R^7$ Wasserstoff oder Methyl bedeutet, wobei das Stickstoffatom eine positive Ladung trägt, oder $R^7$ nicht vorhanden ist;

$Z^5$ Wasserstoff oder eine Gruppe gemäss Definition für $Z^1$ ist und

n 1 oder 2 bedeutet;

oder $Z^2$ und $Z^5$ mit dem Stickstoffatom einen gegebenenfalls ein weiteres Heteroatom enthaltend 3- bis 7-gliedrigen heterocyclischen Ring bilden können,

mit der Massgabe, dass, wenn das Salz der Formel Ia nicht vorhanden ist und $R^1$ Wasserstoff oder eine negative Ladung, $R^2$ Wasserstoff und zwei der Reste $Z^1$, $Z^2$ und $Z^3$ Wasserstoff bedeuten, der andere Rest weder Alkyl noch Alkenyl mit mehr als 7 Kohlenstoffatomen sein kann und

mit der weiteren Massgabe, dass Verbindungen mit den Formeln

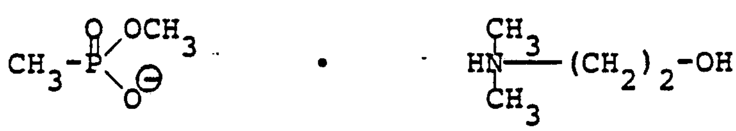

und

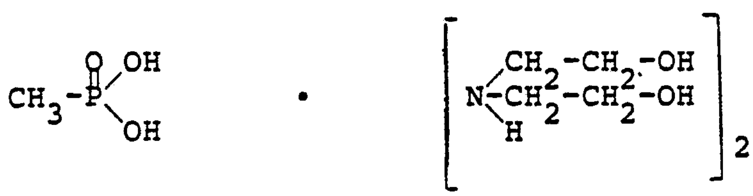

ausgenommen sind.

2. Aminsalze nach Anspruch 1, entsprechend der Formel Ia

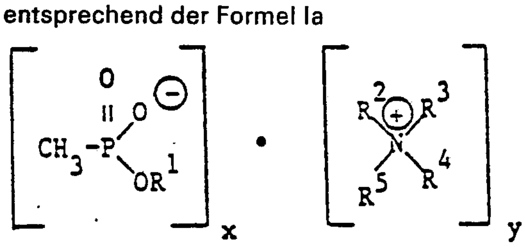

(Ia)

worin x, y, $R^1$, $R^2$, $R^3$, $R^4$ und $R^5$ die in Anspruch 1 angegebenen Bedeutungen haben.

3. Aminsalze nach Anspruch 1, entsprechend der Formel Ib

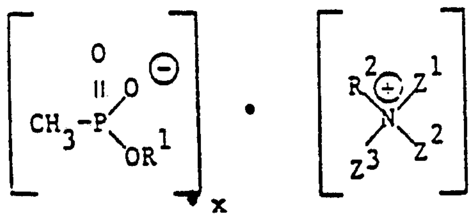

(Ib)

worin x, y, $R^1$, $R^2$, $Z^1$, $Z^2$ und $Z^3$ die in Anspruch 1 angegebenen Bedeutungen haben.

4. Aminsalze nach Anspruch 1, worin $R^1$ Methyl bedeutet.

5. Aminsalze nach Anspruch 1, worin $R^2$ in der Formel Ia Methyl sowie $R^3$ und $R^4$ Hydroxyäthyl bedeuten.

6. Aminsalze nach Anspruch 1, worin $R^1$ und $R^2$ Methyl bedeuten.

7. Aminsalze nach Anspruch 1, dadurch gekennzeichnet, dass im Fall von Substitution der Hydroxylgruppen in $R^3$, $R^4$ und $R^5$ durch eine Oxyalkylenkette, die Kette die Formel

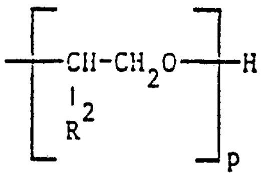

oder

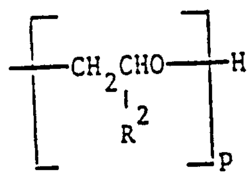

aufweist, worin

$R^2$ Wasserstoff oder Methyl und

p eine ganze Zahl von 1 bis 10 bedeuten.

8. Verfahren zur Herstellung von Aminsalzen nach Anspruch 1, dadurch gekennzeichnet, dass man Methylphosphonsäure bzw. deren Ester der Formel III

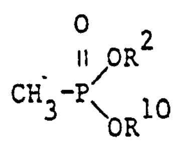

(III)

worin

$R^2$ die in Anspruch 1 angegebene Bedeutung hat und

$R^{10}$ für Wasserstoff oder Methyl steht,

mit einem Amin der Formel IV und/oder V

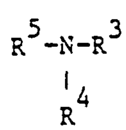

(IV)

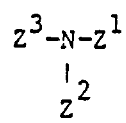

(V)

worin $R^3$, $R^4$, $R^5$, $Z^1$, $Z^2$ und $Z^3$ die in Anspruch 1 angegebenen Bedeutungen haben, in einem wässrigen oder organischen Lösungsmittel sowie gegebenenfalls in einer Inertgasatmosphäre, und nötigenfalls unter Erhitzen, um die Salzbildung zu veranlassen, umsetzt.

9. Verfahren nach Anspruch 8, dadurch gekennzeichnet, dass als Verbindung der Formel III Dimethylmethylphosphonat vorliegt, und die Reaktion durch Erhitzen auf Temperaturen bis zu 200°C durchgeführt wird.

10. Verwendung von Aminsalzen der Formel Ia und/oder Ib nach Anspruch 1 als Flammhemmstoffe für Polyurethane und Polyisocyanurate.

11. Polyurethane bzw. Polyisocyanurate enthaltend flammhemmende Aminsalzen der Formel Ia und/oder Ib

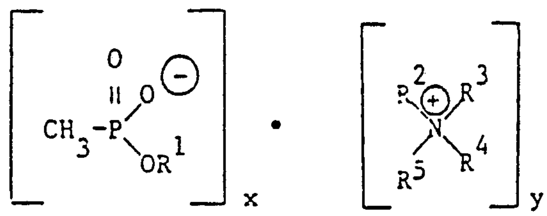

(Ia)

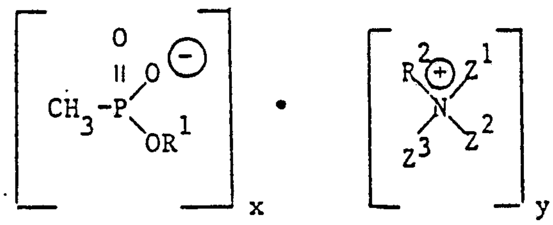

(Ib)

worin

x und y solche ganzen Zahlen sind, dass die Anzahl negativer und positiver Ladungen gleich ist und

$R^1$ Wasserstoff, Methyl oder eine negative Ladung bedeutet,

$R^2$ Wasserstoff oder Methyl,

$R^3$ eine durch 1 - 3 gegebenenfalls durch eine Oxyalkylenkette veretherte Hydroxylgruppen substituierte $C_2$-$C_4$-Alkylgruppen bedeuten, wobei sich nicht mehr als eine Hydroxylgruppe an jeweils einem Kohlenstoffatom befindet,

$R^4$ und $R^5$ gleich oder verschieden sind und dieselbe Bedeutung wie $R^3$ haben können oder Wasserstoff, eine $C_1$-$C_4$-Alkyl-, Phenyl-, Benzylgruppe oder eine am aromatischen Ring durch eine $C_1$-$C_{12}$-Alkylgruppe, eine gegebenenfalls durch eine Oxyalkylenkette veretherte Hydroxylgruppe und/oder 1 - 3 Halogenatome substituierte Phenyl- oder Benzylgruppe bedeuten

oder $R^5$ eine Gruppe der Formel II

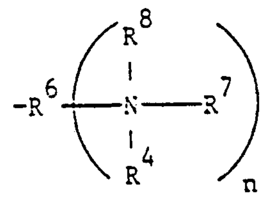

(II)

bedeutet, worin $R^6$ eine $C_2$-$C_4$-Alkylen-, Phenylen-, Xylylen- oder Diphenylmethangruppe oder eine Gruppe der Formel

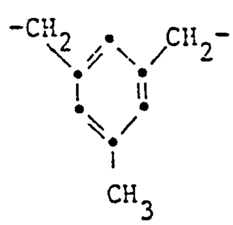

oder

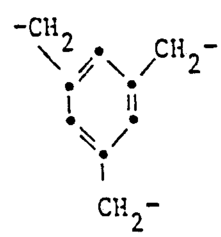

ist, wobei aromatische Ringe in einer $R^6$-Gruppe gegebenenfalls durch eine $C_1$-$C_{12}$-Alkylgruppe, eine gegebenenfalls durch eine Oxyalkylenkette veretherte Hydroxylgruppe und/oder 1 - 3 Halogenatome substituiert sein können, und

$R^7$ Wasserstoff oder Methyl bedeutet, wobei das Stickstoffatom eine positive Ladung trägt, oder $R^7$ nicht vorhanden ist;

$R^8$ Wasserstoff ist oder dieselbe Bedeutung wie $R^3$ hat und

n 1 oder 2 bedeutet

oder $R^4$ und $R^5$ gegebenenfalls zusammen mit dem Stickstoffatom einen gegebenenfalls ein Sauerstoffatom enthaltenden 5- oder 6-gliedrigen heterocyclischen Ring bilden; sowie

$Z^1$, $Z^2$ und $Z^3$ gleich oder verschieden sind und Wasserstoff, eine gerad- oder verzweigtkettige $C_1$-$C_{12}$-Alkyl- bzw. $C_3$-$C_{12}$-Alkenyl- oder Alkinylgruppe, eine $C_4$-$C_{12}$-Cycloalkylgruppe, eine gegebenenfalls durch eine gerad- oder verzweigtkettige $C_1$-$C_4$-Alkylgruppe, eine $C_1$-$C_4$-Alkoxygruppe, Amino, Methylamino, Halogen oder Nitro substituierte Phenyl- oder Naphthylgruppe oder eine $C_7$-$C_{12}$-Aralkylgruppe bedeuten

oder $Z^2$ und $Z^3$ zusammen mit dem sie verknüpfenden Stickstoffatom ein gesättigtes oder ungesättigtes 3- bis 7-gliedriges Ringsystem bilden, welches gegebenenfalls ein weiteres Heteroatom enthält und

gegebenenfalls durch eine gerad- oder verzweigtkettige $C_1$-$C_4$-Alkylgruppe, eine $C_1$-$C_4$-Alkoxygruppe, Amino, Methylamino, eine $C_1$-$C_4$-Aminoalkylgruppe, Halogen oder Nitro substituiert sein kann,

oder $Z^1$, $Z^2$ und $Z^3$ zusammen mit dem sie verknüpfenden Stickstoffatom eine gegebenenfalls ein weiteres Heteroatom enthaltenden 8- bis 12-gliedrigen bicyclischen Ring bilden,

oder $Z^1$ eine Gruppe der Formel

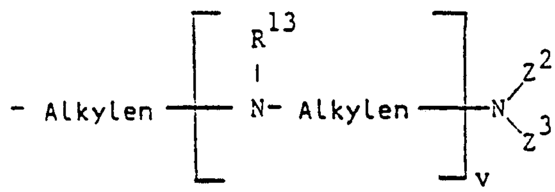

darstellt, worin

Alkylen eine Gruppe mit 2 - 12 Kohlenstoffatomen,

v 0 oder eine ganze Zahl von 1 - 5,

$R^{13}$ Wasserstoff oder eine gerad- bzw. verzweigtkettige $C_1$-$C_{16}$-Alkylgruppe bedeuten und

$Z^2$ sowie $Z^3$ dieselbe Bedeutung wie obenstehend definiert besitzen,

oder $Z^1$ eine Gruppe der Formel

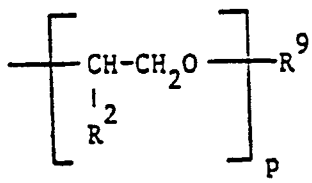

oder

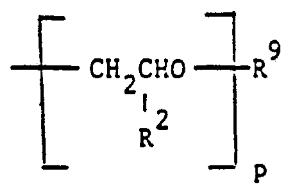

ist, worin

$R^2$ die oben angegebene Bedeutung hat und

$R^9$ eine $C_1$-$C_{12}$-Alkylgruppe und

p eine ganze Zahl von 1 bis 10, vorzugsweise 1 - 4 bedeuten;

oder $Z^3$ eine Gruppe der Formel IIa

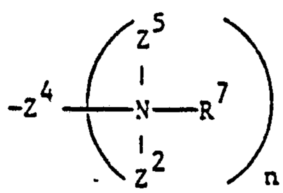

(IIa)

darstellt, worin $Z^4$ eine $C_2$-$C_{12}$-Alkylengruppe, eine Phenylen-, Xylylen- oder Diphenylmethangruppe oder eine Gruppe der Formel

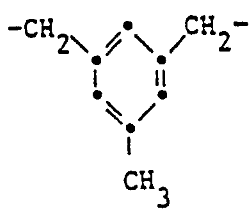

oder

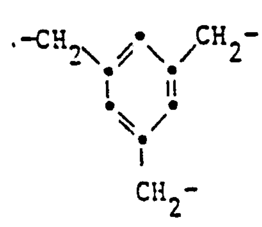

bedeutet, wobei aromatische Ringe in einer $Z^4$-Gruppe gegebenenfalls durch eine $C_1$-$C_{12}$-Alkylgruppe, Hydroxyl und/oder 1 - 3 Halogenatome substituiert sein können, und

$R^7$ Wasserstoff oder Methyl bedeutet, wobei das Stickstoffatom eine positive Ladung trägt, oder $R^7$ nicht vorhanden ist;

$Z^5$ Wasserstoff oder eine Gruppe gemäss Definition für $Z^1$ ist und

n 1 oder 2 bedeutet;

oder $Z^2$ und $Z^5$ mit dem Stickstoffatom einen gegebenenfalls ein weiteres Heteroatom enthaltenden 3- bis 7-gliedrigen heterocyclischen Ring bilden können, mit der Massgabe, dass die Verbindung mit der Formel

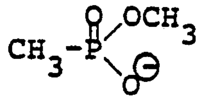 • 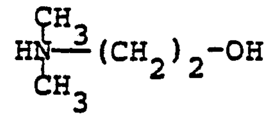

für Polyurethane ausgenommen ist.

**Claims**

1. An amine salt of a phosphonic acid of the general formula Ia and/or Ib

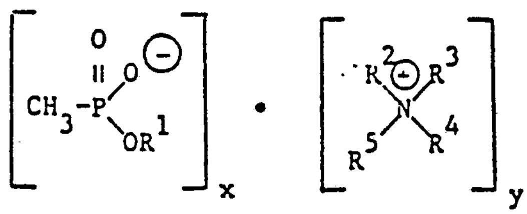

(Ia)

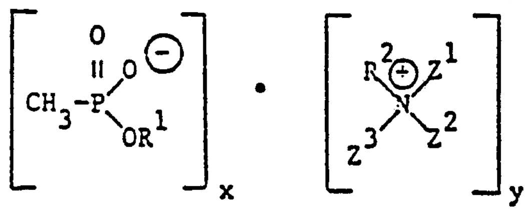

(Ib)

in which

x and y are integers such that the number of negative and positive charges are the same and

$R^1$ is hydrogen, methyl or a negative charge,

$R^2$ is hydrogen or methyl,

$R^3$ is a $C_2$-$C_4$alkyl group substituted by 1 - 3 hydroxyl groups which may be etherified by an oxyalkylene chain, there being not more than one hydroxyl group on any one carbon atom,

$R^4$ and $R^5$ may be the same or different and may be as defined for $R^3$, or hydrogen, a $C_1$-$C_4$alkyl group, a phenyl group, a benzyl group, or a phenyl or benzyl group substituted on the aromatic ring by a $C_1$-$C_{12}$alkyl group, a hydroxyl group which may be etherified by an oxyalkylene chain, and/or 1 - 3 halogen atoms, or

$R^5$ is a group of the formula II

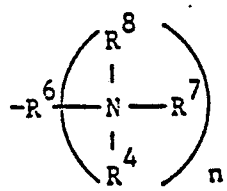

(II)

in which $R^6$ is a $C_2$-$C_4$alkylene group, a phenylene group, a xylylene group, a diphenyl methanegroup or a group of the formula

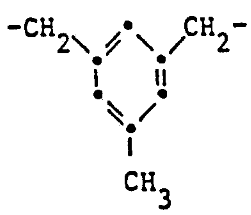

or

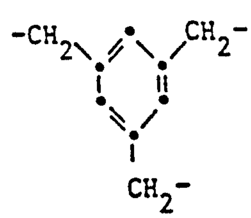

in which aromatic rings in a group $R^6$ may be substituted by a $C_1$-$C_{12}$alkyl group a hydroxyl group which may be etherified by an oxyalkylene chain, and/or 1 - 3 halogen atoms, and

$R^7$ is hydrogen or methyl, in which cases the nitrogen atom carries a positive change, or $R^7$ is absent;

$R^8$ is hydrogen or as defined for $R^3$ and

n is 1 or 2; or $R^4$ and $R^5$, if desired together with the nitrogen, form a 5 or 6 membered heterocyclic ring, optionally containing an oxygen atom; and

$Z^1$, $Z^2$ and $Z^3$ are the same or different and are hydrogen, a straight-chain or branched-chain $C_1$-$C_{12}$alkyl group, a straight-chain or branched-chain $C_3$-$C_{12}$alkenyl group, a straight-chain or branched-chain $C_3$-$C_{12}$alkynyl group, a $C_4$-$C_{12}$cycloalkyl group, a phenyl or naphthyl group which may be substituted by a straight-chain or branched-chain $C_1$-$C_4$alkyl group, a $C_1$-$C_4$alkoxy group, amino, methylamino, halogen or nitro, or a $C_7$-$C_{12}$aralkyl group;

or $Z^2$ and $Z^3$ together with the nitrogen atom to which they are attached form a saturated or unsaturated 3 - 7 membered ring system which may optionally contain another hetero atom and may be substituted by a straight-chain or branched-chain $C_1$-$C_4$alkyl group, a $C_1$-$C_4$alkoxy group, amino, methylamino, a $C_1$-$C_4$aminoalkyl group, halogen or nitro;

or $Z^1$, $Z^2$ and $Z^3$ together with the nitrogen atom to which they are attached form an 8 - 12 membered bicyclic ring, optionally containing another hetero atom,

or $Z^1$ is a group of the formula

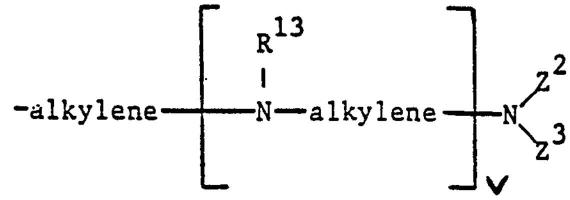

in which

alkylene is a group having 2 - 12 carbon atoms,

v is 0 or an integer from 1 - 5

$R^{13}$ is hydrogen or a straight-chain or branched-chain $C_1$-$C_{16}$alkyl group, and

$Z^2$ and $Z^3$ are as defined above;

or $Z^1$ is a group of the formula

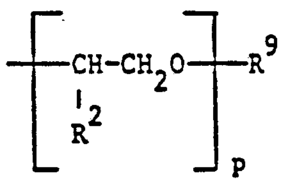

or

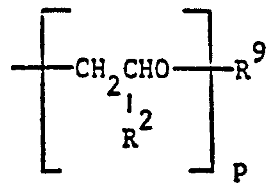

in which

$R^2$ is as defined above,

$R^9$ is a $C_1$-$C_{12}$alkyl group and

p is an integer from 1 to 10, preferably from 1 to 4;

or $Z^3$ is a group of the formula IIa

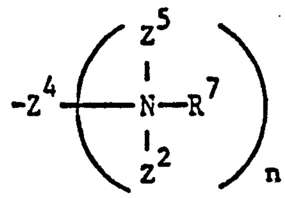

(IIa)

in which $Z^4$ is a $C_2$-$C_{12}$alkylene group, a phenylene group, a xylylene group, a diphenylmethane group or a group of the formula

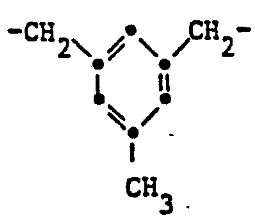

or

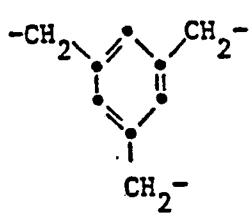

in which aromatic rings in a group $Z^4$ may be substituted by a $C_1$-$C_{12}$alkyl group, hydroxyl and/or 1 - 3 halogen atoms, and

$R^7$ is hydrogen or methyl, in which cases the nitrogen atom carries a positive charge, or $R^7$ is absent;

$Z^5$ is hydrogen or a group as defined for $Z^1$ and

n is 1 or 2;

or $Z^2$ and $Z^5$ may form, with the nitrogen atom, a 3 - 7 membered heterocyclic ring, optionally containing

another hetero atom;

provided that when the salt of the formula Ia is absent and $R^1$ is hydrogen or a negative charge, $R^2$ is hydrogen and two of the radicals $Z^1$, $Z^2$ and $Z^3$ are hydrogen, the other radical cannot be alkyl or alkenyl having more than 7 carbon atoms,

and further provided that compounds having the formulae

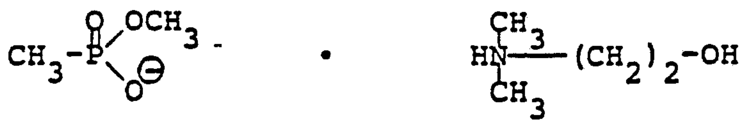

and

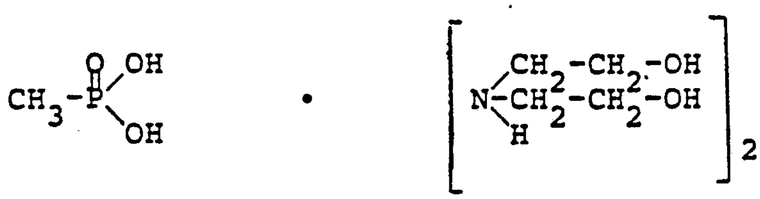

are excepted.

2. An amine salt as claimed in claim 1 which has the formula Ia

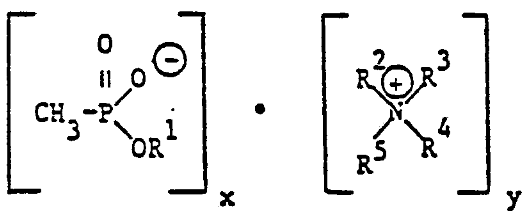 (Ia),

in which x, y, $R^1$, $R^2$, $R^3$, $R^4$ and $R^5$ are as defined in claim 1.

3. An amine salt as claimed in claim 1 which has the formula Ib

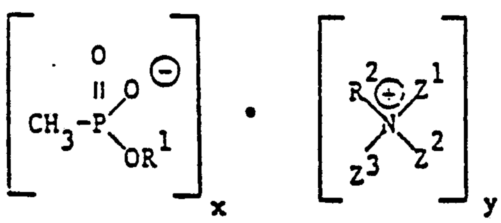 (Ib)

in which x, y, $R^1$, $R^2$, $Z^1$, $Z^2$ and $Z^3$ are as defined in claim 1.

4. An amine salt as claimed in claim 1, in which $R^1$ is methyl.

5. An amine salt as claimed in claim 1, in which $R^2$ in formula Ia is methyl and $R^3$ and $R^4$ are hydroxyethyl.

6. An amine salt as claimed in claim 1, in which $R^1$ and $R^2$ are methyl.

7. An amine salt as claimed in claim 1, wherein, when the hydroxyl groups in $R^3$, $R^4$ and $R^5$ are substituted by an oxyalkylene chain, the chain has the formula

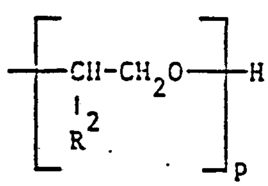 or 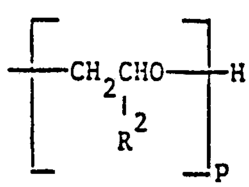

in which

$R^2$ is hydrogen or methyl and

p is an integer from 1 to 10.

8. A process for preparing an amine salt as claimed in claim 1, which comprises reacting methylphosphonic acid or the ester thereof of the formula III

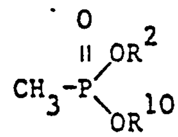

(III)

in which
$R^2$ is as defined in claim 1 and
$R^{10}$ is hydrogen or methyl,
with an amine of the formula IV and/or V

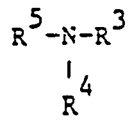

(IV)

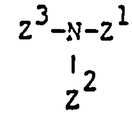

(V)

in which $R^3$, $R^4$, $R^5$, $Z^1$, $Z^2$ and $Z^3$ are as defined in claim 1, in an aqueous or organic solvent and optionally under an inert gas atmosphere, and heating if necessary to cause the salt to form.

9. A process as claimed in claim 8, wherein the compound of formula III is dimethyl methylphosphonate and the reacted is effected by heating at temperatures up to 200°C.

10. The use of amine salts of the formula Ia and/or Ib as claimed in claim 1 as flame retardants for polyurethanes and polyisocyanurates.

11. A polyurethane or polyisocyanurate containing a flame-retardant amine salt of the formula Ia and/or Ib

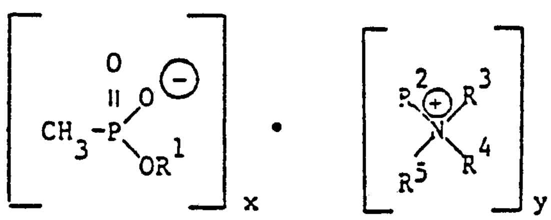

(Ia)

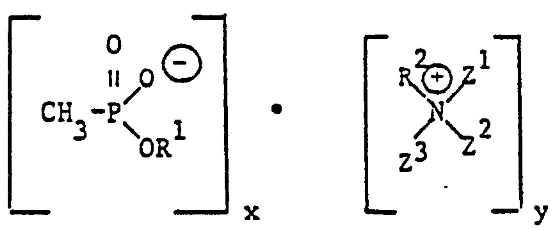

(Ib)

in which
x and y are integers such that the number of negative and positive charges are the same and
$R^1$ is hydrogen, methyl or a negative charge,
$R^2$ is hydrogen or methyl,
$R^3$ is a $C_2$-$C_4$alkyl group substituted by 1 - 3 hydroxyl groups which may be etherified by an oxyalkylene chain, there being not more than one hydroxyl group on any one carbon atom,
$R^4$ and $R^5$ may be the same or different and may be as defined for $R^3$, or hydrogen, a $C_1$-$C_4$alkyl group, a phenyl group, a benzyl group, or a phenyl or benzyl group substituted on the aromatic ring by a $C_1$-$C_{12}$alkyl group, a hydroxyl group which may be etherified by an oxyalkylene chain, and/or 1 - 3 halogen atoms,
or $R^5$ is a group of the formula II

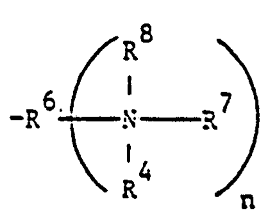

(II)

in which $R^6$ is a $C_2$-$C_4$alkylene group, a phenylene group, a xylylene group, a diphenylmethane group or a group of the formula

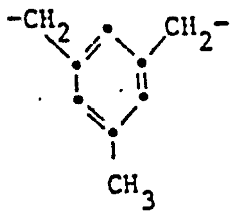

or

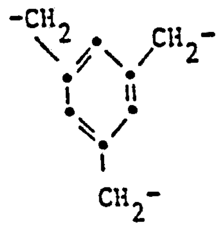

in which aromatic rings in a group $R^6$ may be substituted by a $C_1$-$C_{12}$alkyl group, a hydroxyl group which may be etherified by an oxyalkylene chain, and/or 1 - 3 halogen atoms and

$R^7$ is hydrogen or methyl in which cases the nitrogen atom carries a positive charge or $R^7$ is absent;

$R^8$ is hydrogen or as defined for $R^3$ and

n is 1 or 2;

or $R^4$ and $R^5$, if desired together with the nitrogen, for a 5 or 6 membered heterocyclic ring, optionally containing an oxygen atom;

and $Z^1$, $Z^2$ and $Z^3$ are the same or different and are hydrogen, a straight-chain or branched-chain $C_1$-$C_{12}$alkyl group, a straight-chain or branched-chain $C_3$-$C_{12}$alkenyl group, a straight-chain or branched-chain $C_3$-$C_{12}$alkynyl group, a $C_4$-$C_{12}$cycloalkyl group, a phenyl or naphthyl group which may be substituted by a straight-chain or branched-chain $C_1$-$C_4$alkyl group, a $C_1$-$C_4$alkoxy group, amino, methylamino, halogen or nitro, or a $C_7$-$C_{12}$aralkyl group;

or $Z^2$ and $Z^3$ together with the nitrogen atom to which they are attached form a saturated or unsaturated 3 - 7 membered ring system which may optionally contain another hetero atom and may be substituted by a straight-chain or branched-chain $C_1$-$C_4$alkyl group, a $C_1$-$C_4$alkoxy group, amino, methylamino, a $C_1$-$C_4$aminoalkyl group, halogen or nitro;

or $Z^1$, $Z^2$ and $Z^3$ together with the nitrogen atom to which they are attached form an 8 - 12 membered bicyclic ring optionally containing another hetero atom or

$Z^1$ is a group of the formula

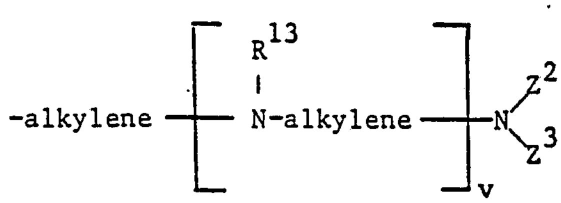

in which

alkylene is a group having 2 - 12 carbon atoms,

v is 0 or an integer from 1 to 5,

$R^{13}$ is hydrogen or a straight-chain or branched-chain $C_1$-$C_{16}$alkyl group, and

$Z^2$ and $Z^3$ are as defined above;

or $Z^1$ is a group of the formula

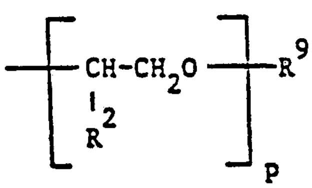

or

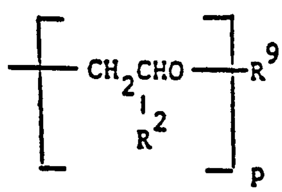

in which

$R^2$ is as defined above,

$R^9$ is a $C_1$-$C_{12}$alkyl group and

p is an integer from 1 to 10, preferably 1 - 4; or $Z^3$ is a group of the formula IIa

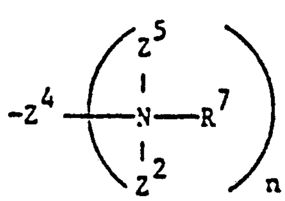

(IIa)

in which $Z^4$ is a $C_2$-$C_{12}$alkylene group, a phenylene group, a xylylene group, a diphenylmethane grop or a group of the formula

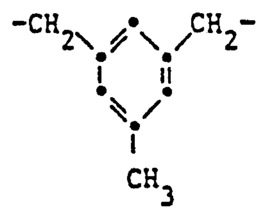

or

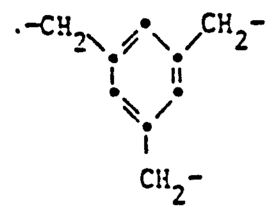

in which aromatic rings in a group $Z^4$ may be substituted by a $C_1$-$C_{12}$alkyl group, hydroxyl and/or 1 - 3 halogen atoms, and

$R^7$ is hydrogen or methyl, in which cases the nitrogen atom carries a positive charge, or $R^7$ is absent;

$Z^5$ is hydrogen or a group as defined for $Z^1$ and

n is 1 or 2;

or $Z^2$ and $Z^5$ may form, with the nitrogen atom, a 3 - 7 membercd heterocyclic ring optionally containing another hetero atom, provided that the compound having the formula

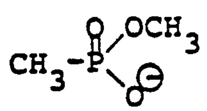

•

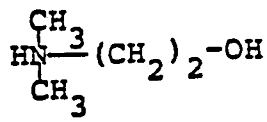

is excepted for polyurethanes.

## Revendications

1. Sels d'amines d'acides phosphoniques, de formule générale Ia et/ou Ib

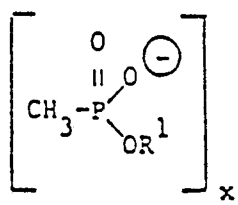

•

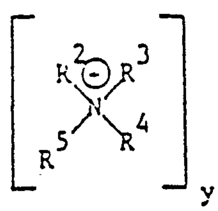

(Ia)

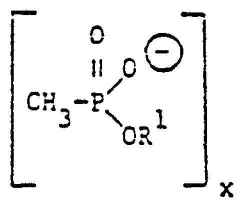

•

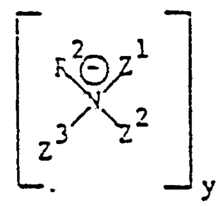

(Ib)

dans lesquelles

x et y sont des nombres entiers tels que le nombre des charges négatives est égal au nombre des charges positives, et

$R^1$ représente un atome d'hydrogène, un groupe méthyle ou une charge négative;

$R^2$ représente un atome d'hydrogène ou un groupe méthyle;

$R^3$ représente un groupe alkyle en $C_2$ à $C_4$, substitué par 1 à 3 fois par des groupes hydroxyles éventuellement éthérifiés par une chaîne oxyalkylène, chaque atome de carbone ne portant pas plus d'un groupe hydroxyle;

$R^4$ et $R^5$ sont identiques ou différents et peuvent avoir le même sens que $R^3$ ou bien représenter un atome d'hydrogène, un groupe alkyle en $C_1$ à $C_4$, un groupe phényle, un groupe benzyle ou un groupe phényle ou benzyle (substitué sur le noyau benzénique par un groupe alkyle en $C_1$ à $C_{12}$, par un groupe hydroxyle éventuellement éthérifié par une chaîne oxyalkylène et/ou par 1 à 3 atomes d'halogène),

ou bien $R^5$ représente un groupe de formule II

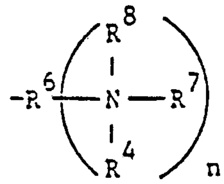 (II)

où $R^6$ représente un groupe alkylène en $C_2$ à $C_4$, un groupe phénylène, xylylène ou diphénylméthane, ou un groupe de formule

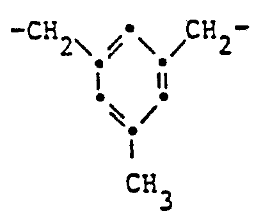 ou 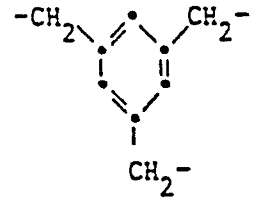

les noyaux aromatiques d'un groupe $R^6$ pouvant éventuellement être substitués par un groupe alkyle en $C_1$ à $C_{12}$, par un groupe hydroxyle éventuellement éthérifié par une chaîne oxyalkylène, et/ou par 1 à 3 atomes d'halogène, et

$R^7$ représente un atome d'hydrogène ou un groupe méthyle, l'atome d'azote portant une charge positive, ou bien $R^7$ n'est pas présent;

$R^8$ représente un atome d'hydrogène ou a le même sens que $R^3$, et

n vaut 1 ou 2,

ou bien $R^4$ et $R^5$ forment, avec l'atome d'azote, un noyau hétérocyclique pentagonal ou hexagonal contenant éventuellement un atome d'oxygène; et

$Z^1$, $Z^2$ et $Z^3$ sont identiques ou différents et représentent chacun un atome d'hydrogène, un groupe alkyle en $C_1$ à $C_{12}$ ou alcényle en $C_3$ ou $C_{12}$ ou alcynyle en $C_3$ à $C_{12}$, linéaire ou ramifiés, un groupe cycloalkyle en $C_4$ à $C_{12}$, un groupe phényle ou naphtyle (éventuellement substitué par un groupe alkyle en $C_1$ à $C_4$, linéaires ou ramifiés, par un groupe alcoxy en $C_1$ à $C_4$, par un groupe amino, méthylamino, halogéno ou nitro) ou un groupe aralkyle en $C_7$ à $C_{12}$,

ou bien $Z^2$ et $Z^3$ forment avec l'atome d'azote auquel ils sont reliés, un système cyclique triangulaire à heptagonal, saturé ou insaturé, qui contient éventuellement un autre hétéroatome et peut éventuellement être substitué par un groupe alkyle en $C_1$ à $C_4$, linéaire ou ramifié, par un groupe alcoxy en $C_1$ à $C_4$, par un groupe amino, méthylamino, par un groupe aminoalkyle en $C_1$ à $C_4$, par de l'halogène ou par un groupe nitro;

ou bien $Z^1$, $Z^2$ et $Z^3$, pris avec l'atome d'azote auquel ils sont reliés, forment un noyau bicyclique comportant 8 à 12 chaînons et contenant éventuellement un autre hétéroatome,

ou bien $Z^1$ représente un groupe de formule

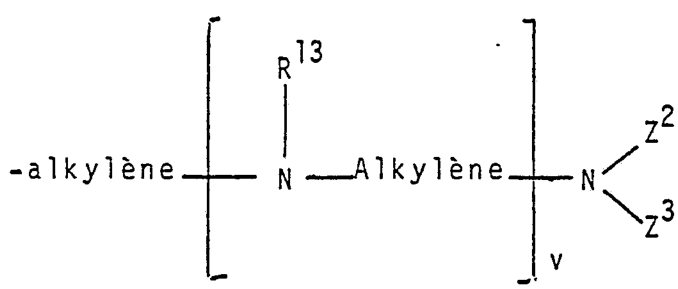

où

alkylène représente un groupe ayant 2 à 12 atomes de carbone,

v est nul ou est un nombre entier valant 1 à 5,

$R^{13}$ représente un atome d'hydrogène ou un groupe alkyle en $C_1$ à $C_{16}$, linéaire ou ramifié, et

$Z^2$ et $Z^3$ ont le même sens que celui défini ci-dessus,

ou bien $Z^1$ représente un groupe de formule

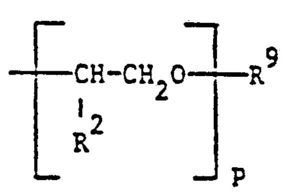 ou 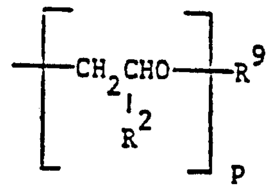

$R^2$ a le sens indiqué ci-dessus et

$R^9$ représente un groupe alkyle en $C_1$ à $C_{12}$, et
p est un nombre entier valant 1 à 10, de préférence 1 à 4;
ou bien $Z^3$ représente un groupe de formule IIa

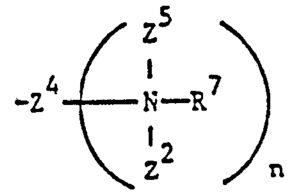 (IIa)

dans laquelle $Z^4$ représente un groupe alkylène en $C_2$ à $C_{12}$, un groupe phénylène, xylylène ou diphénylméthane, ou un groupe de formule

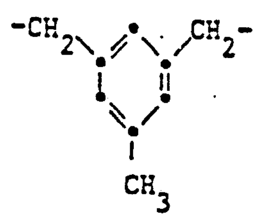 ou 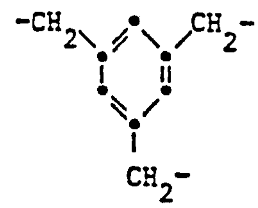

où les noyaux aromatiques d'un groupe $Z^4$ peuvent éventuellement être substitués par un groupe alkyle en $C_1$ à $C_{12}$, par un groupe hydroxyle et/ou par 1 à 3 atomes d'halogène, et

$R^7$ représente un atome d'hydrogène ou un groupe méthyle, l'atome d'azote portant une charge positive, ou bien $R^7$ n'est pas présent;

$Z^5$ représente un atome d'hydrogène ou un groupe correspondant à la définition de $Z^1$ et

n vaut 1 ou 2;

ou bien $Z^2$ et $Z^5$ peuvent former, avec l'atome d'azote, un système hétérocyclique comportant 3 à 7 chaînons et contenant éventuellement un autre hétéroatome,

à la condition que, lorsque le sel de formule Ia n'est pas présent et que $R^1$ représente un atome d'hydrogène ou une charge négative, que $R^2$ représente un atome d'hydrogène et que deux des restes $Z^1$, $Z^2$ et $Z^3$ représentent de l'hydrogène, l'autre reste ne peut être ni un groupe alkyle ni un groupe alcényle contenant plus de 7 atomes de carbone, et

à la condition d'exclure les composés répondant aux formules

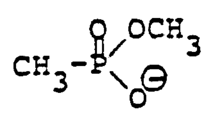 • 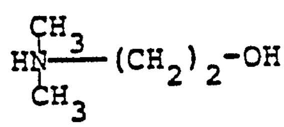

et

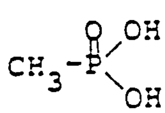 • 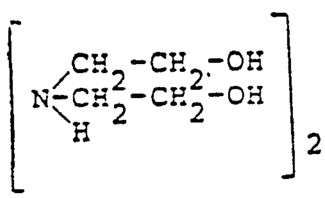

2. Sels d'amines selon la revendication 1, répondant à la formule Ia

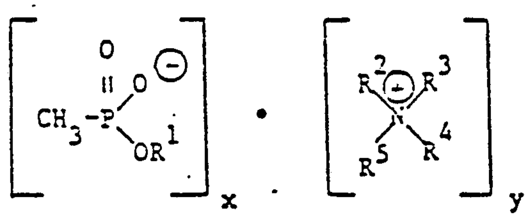

(I a)

dans laquelle x, y, $R^1$, $R^2$, $R^3$, $R^4$ et $R^5$ ont les sens indiqués à la revendication 1.

3. Sels d'amines selon la revendication 1, répondant à la formule Ib

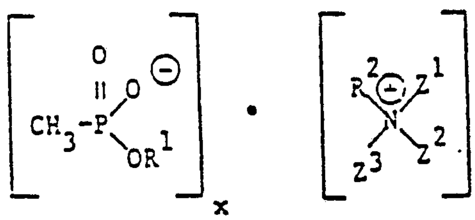

(I b)

dans laquelle x, y, $R^1$, $R^2$, $Z^1$, $Z^2$, $Z^3$ ont les sens indiqués à la revendication 1.

4. Sels d'amines selon la revendication 1, dans lesquels $R^1$ représente un groupe méthyle.

5. Sels d'amines selon la revendication 1, dans lesquels $R^2$, dans la formule Ia, représente un groupe méthyle, et $R^3$, $R^4$ représentent les groupes hydroxyéthyles.

6. Sels d'amines selon la revendication 1, dans lesquels $R^1$ et $R^2$ représentent chacun un groupe méthyle.

7. Sels d'amines selon la revendication 1, caractérisés en ce qu'en cas de substitution des groupes hydroxyles de $R^3$, $R^4$ et $R^5$ par une chaîne oxyalkylène, la chaîne présente la formule

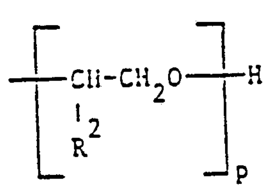

ou

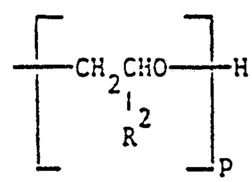

$R^2$ représente un atome d'hydrogène ou un groupe méthyle et
p est un nombre entier valant 1 à 10.

8. Procédé pour préparer des sels d'amines selon la revendication 1, caractérisé en ce qu'on fait réagir de l'acide méthanephosphonique ou ses esters de formule III

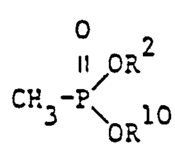

(III)

(dans laquelle $R^2$ a le sens indique à la revendication 1 et $R^{10}$ représente un atome d'hydrogène ou un groupe méthyle) avec une amine de formule IV et/ou V

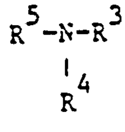

(IV)

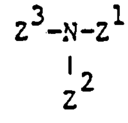

(V)

(dans lesquelles $R^3$, $R^4$, $R^5$, $Z^1$, $Z^2$ et $Z^3$ ont les sens indiqués à la revendication 1) dans un solvant aqueux ou organique et éventuellement en atmosphère de gaz inerte et en opérant, en cas de besoin, avec chauffage, pour permettre la formation des sels.

9. Procédé selon la revendication 8, caractérisé en ce que le méthane phosphonate de diméthyle est présent comme composé de formule III, et en ce qu'on conduit la réaction par chauffage à des températures allant jusqu'à 200° C.

10. Utilisation de sels d'amines de formule (Ia) et/ou (Ib) selon la revendication 1, comme agent retardant la

propagation d'une flamme (agent d'ignifugation) pour des polyuréthannes et des polyisocyanurates.

11. Polyuréthannes ou polyisocyanurates contenant des sels d'amines, à rôle de retard de propagation de flamme ou d'ignifugation, ces sels répondant à la formule Ia et/ou Ib

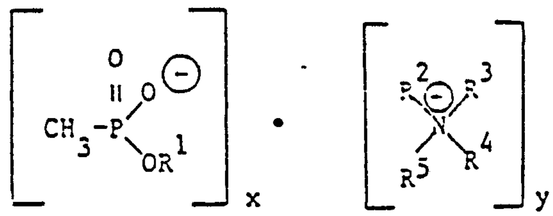

(Ia)

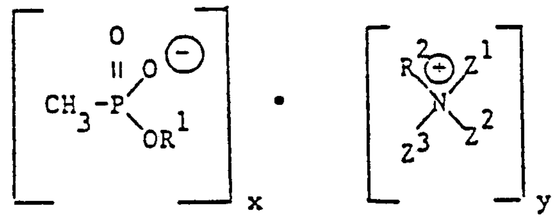

(Ib)

dans lesquelles

x et y sont des nombres entiers tels que le nombre des charges négatives est égal au nombre des charges positives, et

$R^1$ représente un atome d'hydrogène, un groupe méthyle ou une charge négative;

$R^2$ représente un atome d'hydrogène ou un groupe méthyle;

$R^3$ représente un groupe alkyle en $C_2$ à $C_4$, substitué par 1 à 3 fois par des groupes hydroxyles éventuellement éthérifiés par une chaîne oxyalkylène, chaque atome de carbone ne portant pas plus d'un groupe hydroxyle;

$R^4$ et $R^5$ sont identiques ou différents et peuvent avoir le même sens que $R^3$ ou bien représenter un atome d'hydrogène, un groupe alkyle en $C_1$ à $C_4$, un groupe phényle, un groupe benzyle ou un groupe phényle ou benzyle (substitué sur le noyau benzénique par un groupe alkyle en $C_1$ à $C_{12}$, par un groupe hydroxyle éventuellement éthérifié par une chaîne oxyalkylène et/ou par 1 à 3 atomes d'halogène),

ou bien $R^5$ représente un groupe de formule II

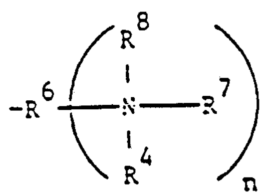

(II)

où $R^6$ représente un groupe alkylène en $C_2$ à $C_4$, un groupe phénylène, xylylène ou diphénylméthane, ou un groupe de formule

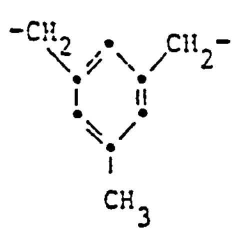

ou

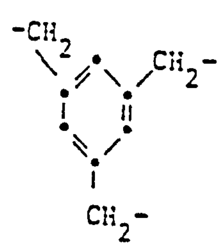

les noyaux aromatiques d'un groupe $R^6$ pouvant éventuellement être substitués par un groupe alkyle en $C_1$ à $C_{12}$, par un groupe hydroxyle éventuellement éthérifié par une chaîne oxyalkylène, et/ou par 1 à 3 atomes d'halogène, et

$R^7$ représente un atome d'hydrogène ou un groupe méthyle, l'atome d'azote portant une charge positive, ou bien $R^7$ n'est pas présent;

$R^8$ représente un atome d'hydrogène ou a le même sens que $R^3$, et

n vaut 1 ou 2,

ou bien $R^4$ et $R^5$ forment, avec l'atome d'azote, un noyau hétérocyclique pentagonal ou hexagonal contenant éventuellement un atome d'oxygène; et

$Z^1$, $Z^2$ et $Z^3$ sont identiques ou différents et représentent chacun un atome d'hydrogène, un groupe alkyle en $C_1$ à $C_{12}$ ou alcényle en $C_3$ à $C_{12}$ ou alcynyle en $C_3$ à $C_{12}$, linéaire ou ramifiés, un groupe cycloalkyle en $C_4$ à $C_{12}$, un groupe phényle ou naphtyle (éventuellement substitué par un groupe alkyle en $C_1$ à $C_4$, linéaires ou ramifiés, par un groupe alcoxy en $C_1$ à $C_4$, par un groupe amino, methylamino, halogéno ou nitro) ou un groupe aralkyle en $C_7$ à $C_{12}$,

ou bien $Z^2$ et $Z^3$ forment avec l'atome d'azote auquel ils sont reliés, un système cyclique triangulaire à heptagonal, saturé ou insaturé, qui contient éventuellement un autre hétéroatome et peut éventuellement être substitué par un groupe alkyle en $C_1$ à $C_4$, linéaire ou ramifié, par un groupe alcoxy en $C_1$ à $C_4$, par un groupe amino, méthylamino, par un groupe aminoalkyle en $C_1$ à $C_4$, par de l'halogène ou par un groupe nitro;

ou bien $Z^1$, $Z^2$ et $Z^3$, pris avec l'atome d'azote auquel ils sont reliés, forment un noyau bicyclique comportant 8 à 12 chaînons et contenant éventuellement un autre hétéroatome,

ou bien $Z^1$ représente un groupe de formule

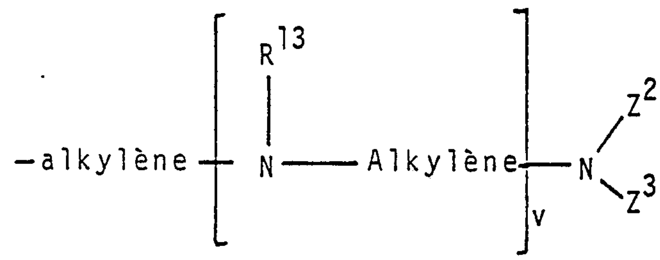

alkylène représente un groupe ayant 2 à 12 atomes de carbone,
v est nul ou est un nombre entier valant 1 à 5,
$R^{13}$ représente un atome d'hydrogène ou un groupe alkyle en $C_1$ à $C_{16}$, linéaire ou ramifié, et
$Z^2$ et $Z^3$ ont le même sens que celui défini ci-dessus
ou bien $Z^1$ représente un groupe de formule

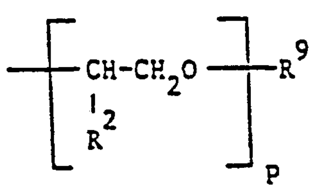

ou

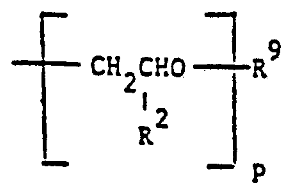

$R^2$ a le sens indiqué ci-dessus et
$R^9$ représente un groupe alkyle en $C_1$ à $C_{12}$, et
p est un nombre entier valant 1 à 10, de préférence 1 à 4;
ou bien $Z^3$ représente un groupe de formule IIa

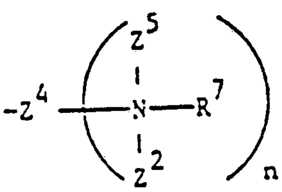

(IIa)

dans laquelle $Z^4$ représente un groupe alkylène en $C_2$ à $C_{12}$, un groupe phénylène, xylylène ou diphénylméthane, ou un groupe de formule

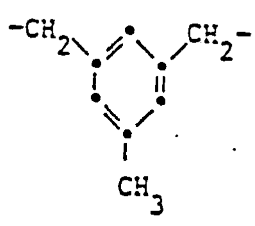

ou

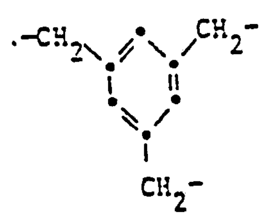

où les noyaux aromatiques d'un groupe $Z^4$ peuvent éventuellement être substitués par un groupe alkyle en $C_1$ à $C_{12}$, par un groupe hydroxyle et/ou par 1 à 3 atomes d'halogène, et

$R^7$ représente un atome d'hydrogène ou un groupe méthyle, l'atome d'azote portant une charge positive, ou bien $R^7$ n'est pas présent;

$Z^5$ représente un atome d'hydrogène ou un groupe correspondant à la définition de $Z^1$ et
n vaut 1 ou 2;
ou bien $Z^2$ et $Z^5$ peuvent former, avec l'atome d'azote, un système hétérocyclique comportant 3 à 7 chaînons et contenant éventuellement un autre hétéroatome,
à la condition que, lorsque le sel de formule Ia n'est pas présent et que $R^1$ représente un atome d'hydrogène ou une charge négative, que $R^2$ représente un atome d'hydrogène et que deux des restes $Z^1$, $Z^2$ et $Z^3$ représentent de l'hydrogène, l'autre reste ne peut être ni un groupe alkyle ni un groupe alcényle contenant plus de 7 atomes de carbone, et
à la condition d'exclure, pour les polyuréthannes, le composé de formule

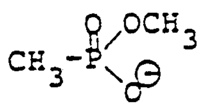 • 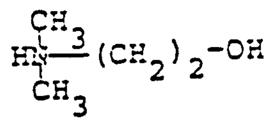 .